# EUROPEAN PATENT SPECIFICATION

(11) **EP 0 669 979 B1**
(45) Date of publication and mention of the grant of the patent: **12.10.2005**
(21) Application number: 94901289.2
(22) Date of filing: 04.11.1993
(51) Int. Cl.: C12N 15/12, C07K 14/00, C12P 21/08, C12N 5/10, C12N 15/62, A61K 38/00, A61K 39/395, C12Q 1/68

(54) **C-C CKR-1, C-C CHEMOKINE RECEPTOR**
C-C CKR-1, EIN C-C CHEMOKIN REZEPTOR
RECEPTEUR DE LA CHEMOKINE C-C (C-C CKR-1)

(30) Priority: 10.11.1992 US 974025
(43) Date of publication of application: 06.09.1995
(73) Proprietor: GENENTECH, INC., California 94080 (US)
(72) Inventor: HORUK, Richard, Belmont, CA 94002 (US); NEOTE, Kuldeep, East Lyme, CT 06333 (US); SCHALL, Thomas, Menlo Park, CA 94025 (US)
(74) Representative: Ellis, Edward Lovell
(86) International application number: PCT/US1993/010672
(87) International publication number: WO 1994/011504

(56) References cited:
- WO-A-92/17497
- WO-A-92/18641
- CELL vol. 72 , 12 February 1993 , CAMBRIDGE, NA US pages 415 - 425 NEOTE K;DIGREGORIO D;MAK JY;HORUK R;SCHALL TJ 'Molecular cloning, functional expression, and signaling'

### SEQUENCE LISTING

(1) GENERAL INFORMATION:
   (i) APPLICANT: GENENTECH, INC.
   (ii) TITLE OF INVENTION: CC-CHEMOKINE RECEPTOR
   (iii) NUMBER OF SEQUENCES: 11
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 5.25 inch, 360 Kb floppy disk
      (B) COMPUTER: IBM PC compatible
      (C) OPERATING SYSTEM: PC-DOS/MS-DOS
      (D) SOFTWARE: patin (Genentech)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: Fitts, Renee A.
      (B) REGISTRATION NUMBER: 35,136
      (C) REFERENCE/DOCKET NUMBER: 806
   (ix) TELECOMMUNICATION INFORMATION:
      (A) TELEPHONE: 415/225-1489
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 355 amino acids
      (B) TYPE: amino acid
      (D) TOPOLOGY: linear
   (xi) SEQUENCE DESCRIPTION: SEQ ID NO:1:
   Met Glu Thr Pro Asn Thr Thr Glu Asp Tyr Asp Thr Thr Thr Glu
    1               5                  10                  15
   Phe Asp Tyr Gly Asp Ala Thr Pro Cys Gln Lys Val Asn Glu Arg
                    20                  25                  30
   Ala Phe Gly Ala Gln Leu Leu Pro Pro Leu Tyr Ser Leu Val Phe
                    35                  40                  45
   Val Ile Gly Leu Val Gly Asn Ile Leu Val Val Leu Val Leu Val
                    50                  55                  60
   Gln Tyr Lys Arg Leu Lys Asn Met Thr Ser Ile Tyr Leu Leu Asn
                    65                  70                  75
   Leu Ala Ile Ser Asp Leu Leu Phe Leu Phe Thr Leu Pro Phe Trp
                    80                  85                  90
   Ile Asp Tyr Lys Leu Lys Asp Asp Trp Val Phe Gly Asp Ala Met
                    95                 100                 105
   Cys Lys Ile Leu Ser Gly Phe Tyr Tyr Thr Gly Leu Tyr Ser Glu
                   110                 115                 120
   Ile Phe Phe Ile Ile Leu Leu Thr Ile Asp Arg Tyr Leu Ala Ile
                   125                 130                 135
   Val His Ala Val Phe Ala Leu Arg Ala Arg Thr Val Thr Phe Gly
                   140                 145                 150
   Val Ile Thr Ser Ile Ile Ile Trp Ala Leu Ala Ile Leu Ala Ser
                   155                 160                 165
   Met Pro Gly Leu Tyr Phe Ser Lys Thr Gln Trp Glu Phe Thr His
                   170                 175                 180
   His Thr Cys Ser Leu His Phe Pro His Glu Ser Leu Arg Glu Trp
                   185                 190                 195
   Lys Leu Phe Gln Ala Leu Lys Leu Asn Leu Phe Gly Leu Val Leu
                   200                 205                 210
   Pro Leu Leu Val Met Ile Ile Cys Tyr Thr Gly Ile Ile Lys Ile
                   215                 220                 225
   Leu Leu Arg Arg Pro Asn Glu Lys Lys Ser Lys Ala Val Arg Leu
                   230                 235                 240
   Ile Phe Val Ile Met Ile Ile Phe Phe Leu Phe Trp Thr Pro Tyr
                   245                 250                 255
   Asn Leu Thr Ile Leu Ile Ser Val Phe Gln Asp Phe Leu Phe Thr
                   260                 265                 270
   His Glu Cys Glu Gln Ser Arg His Leu Asp Leu Ala Val Gln Val
                   275                 280                 285
   Thr Glu Val Ile Ala Tyr Thr His Cys Cys Val Asn Pro Val Ile
                   290                 295                 300
   Tyr Ala Phe Val Gly Glu Arg Phe Arg Lys Tyr Leu Arg Gln Leu
   305 310 315
   Phe His Arg Arg Val Ala Val His Leu Val Lys Trp Leu Pro Phe
   320 325 330
   Leu Ser Val Asp Arg Leu Glu Arg Val Ser Ser Thr Ser Pro Ser
   335 340 345
   Thr Gly Glu His Glu Leu Ser Ala Gly Phe
   350 355
(2) INFORMATION FOR SEQ ID NO:2:
   (i) SEQUENCE CHARACTERISTICS:
      (A) LENGTH: 352 amino acids
      (B) TYPE: amino acid
      (D) TOPOLOGY: linear
   (xi) SEQUENCE DESCRIPTION: SEQ ID NO:2:
   Met Glu Gly Ile Ser Ile Tyr Thr Ser Asp Asn Tyr Thr Glu Glu
    1               5                  10                  15
   Met Gly Ser Gly Asp Tyr Asp Ser Met Lys Glu Pro Cys Phe Arg
                   20                  25                  30
   Glu Glu Asn Ala Asn Phe Asn Lys Ile Phe Leu Pro Thr Ile Tyr
                   35                  40                  45
   Ser Ile Ile Phe Leu Thr Gly Ile Val Gly Asn Gly Leu Val Ile
                   50                  55                  60
   Leu Val Met Gly Tyr Gln Lys Lys Leu Arg Ser Met Thr Asp Lys
                   65                  70                  75
   Tyr Arg Leu His Leu Ser Val Ala Asp Leu Leu Phe Val Ile Thr
                   80                  85                  90
   Leu Pro Phe Trp Ala Val Asp Ala Val Ala Asn Trp Tyr Phe Gly
                   95                 100                 105
   Asn Phe Leu Cys Lys Ala Val His Val Ile Tyr Thr Val Asn Leu
                  110                 115                 120
   Tyr Ser Ser Val Leu Ile Leu Ala Phe Ile Ser Leu Asp Arg Tyr
                  125                 130                 135
   Leu Ala Ile Val His Ala Thr Asn Ser Gln Arg Pro Arg Lys Leu
                  140                 145                 150
   Leu Ala Glu Lys Val Val Tyr Val Gly Val Trp Ile Pro Ala Leu
                  155                 160                 165
   Leu Leu Thr Ile Pro Asp Phe Ile Phe Ala Asn Val Ser Glu Ala
                  170                 175                 180
   Asp Asp Arg Tyr Ile Cys Asp Arg Phe Tyr Pro Asn Asp Leu Trp
                   185                 190                 195
   Val Val Val Phe Gln Phe Gln His Ile Met Val Gly Leu Ile Leu
                   200                 205                 210
   Pro Gly Ile Val Ile Leu Ser Cys Tyr Cys Ile Ile Ile Ser Lys
                   215                 220                 225
   Leu Ser His Ser Lys Gly His Gln Lys Arg Lys Ala Leu Lys Thr
                   230                 235                 240
   Thr Val Ile Leu Ile Leu Ala Phe Phe Ala Cys Trp Leu Pro Tyr
                   245                 250                 255
   Tyr Ile Gly Ile Ser Ile Asp Ser Phe Ile Leu Leu Glu Ile Ile
                   260                 265                 270
   Lys Gln Gly Cys Glu Phe Glu Asn Thr Val His Lys Trp Ile Ser
                   275                 280                 285
   Ile Thr Glu Ala Leu Ala Phe Phe His Cys Cys Leu Asn Pro Ile
                   290                 295                 300
   Leu Tyr Ala Phe Leu Gly Ala Lys Phe Lys Thr Ser Ala Gln His
                   305                 310                 315
   Ala Leu Thr Ser Val Ser Arg Gly Ser Ser Leu Lys Ile Leu Ser
                   320                 325                 330
   Lys Gly Lys Arg Gly Gly His Ser Ser Val Ser Thr Glu Ser Glu
                   335                 340                 345
   Ser Ser Ser Phe His Ser Ser
                   350     352
(2) INFORMATION FOR SEQ ID NO:3:
   (i) SEQUENCE CHARACTERISTICS:
      (A) LENGTH: 350 amino acids
      (B) TYPE: amino acid
      (D) TOPOLOGY: linear
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 355 amino acids
      (B) TYPE: amino acid
      (D) TOPOLOGY: linear
   (xi) SEQUENCE DESCRIPTION: SEQ ID NO:4:
   Met Glu Ser Asp Ser Phe Glu Asp Phe Trp Lys Gly Glu Asp Leu
     1               5                  10                  15
   Ser Asn Tyr Ser Tyr Ser Ser Thr Leu Pro Pro Phe Leu Leu Asp
                    20                  25                  30
   Ala Ala Pro Cys Glu Pro Glu Ser Leu Glu Ile Asn Lys Tyr Phe
                    35                  40                  45
   Val Val Ile Ile Tyr Ala Leu Val Phe Leu Leu Ser Leu Leu Gly
                    50                  55                  60
   Asn Ser Leu Val Met Leu Val Ile Leu Tyr Ser Arg Val Gly Arg
                    65                  70                  75
   Ser Val Thr Asp Val Tyr Leu Leu Asn Leu Ala Leu Ala Asp Leu
                    80                  85                  90
   Leu Phe Ala Leu Thr Leu Pro Ile Trp Ala Ala Ser Lys Val Asn
                    95                 100                 105
   Gly Trp Ile Phe Gly Thr Phe Leu Cys Lys Val Val Ser Leu Leu
                   110                 115                 120
   Lys Glu Val Asn Phe Tyr Ser Gly Ile Leu Leu Leu Ala Cys Ile
                   125                 130                 135
   Ser Val Asp Arg Tyr Leu Ala Ile Val His Ala Thr Arg Thr Leu
                   140                 145                 150
   Thr Gln Lys Arg Tyr Leu Val Lys Phe Ile Cys Leu Ser Ile Trp
                   155                 160                 165
   Gly Leu Ser Leu Leu Leu Ala Leu Pro Val Leu Leu Phe Arg Arg
                   170                 175                 180
   Thr Val Tyr Ser Ser Asn Val Ser Pro Ala Cys Tyr Glu Asp Met
                   185                 190                 195
   Gly Asn Asn Thr Ala Asn Trp Arg Met Leu Leu Arg Ile Leu Pro
                   200                 205                 210
   Gln Ser Phe Gly Phe Ile Val Pro Leu Leu Ile Met Leu Phe Cys
                   215                 220                 225
   Tyr Gly Phe Thr Leu Arg Thr Leu Phe Lys Ala His Met Gly Gln
                   230                 235                 240
   Lys His Arg Ala Met Arg Val Ile Phe Ala Val Val Leu Ile Phe
                   245                 250                 255
   Leu Leu Cys Trp Leu Pro Tyr Asn Leu Val Leu Leu Ala Asp Thr
                   260                 265                 270
   Leu Met Arg Thr Gln Val Ile Gln Glu Thr Cys Glu Arg Arg Asn
                   275                 280                 285
   His Ile Asp Arg Ala Leu Asp Ala Thr Glu Ile Leu Gly Ile Leu
                   290                 295                 300
   His Ser Cys Leu Asn Pro Leu Ile Tyr Ala Phe Ile Gly Gln Lys
                   305                 310                 315
   Phe Arg His Gly Leu Leu Lys Ile Leu Ala Ile His Gly Leu Ile
                   320                 325                 330
   Ser Lys Asp Ser Leu Pro Lys Asp Ser Arg Pro Ser Phe Val Gly
                   335                 340                 345
   Ser Ser Ser Gly His Thr Ser Thr Thr Leu
                   350                 355
(2) INFORMATION FOR SEQ ID NO:5:
   (i) SEQUENCE CHARACTERISTICS:
      (A) LENGTH: 350 amino acids
      (B) TYPE: amino acid
      (D) TOPOLOGY: linear
   (xi) SEQUENCE DESCRIPTION: SEQ ID NO:5:
   Met Asn Ser Phe Asn Tyr Thr Thr Pro Asp Tyr Gly His Tyr Asp
   1 5 10 15
   Asp Lys Asp Thr Leu Asp Leu Asn Thr Pro Val Asp Lys Thr Ser
   20 25 30
   Asn Thr Leu Arg Val Pro Asp Ile Leu Ala Leu Val Ile Phe Ala
   35 40 45
   Val Val Phe Leu Val Gly Val Leu Gly Asn Ala Leu Val Val Trp
   50 55 60
   Val Thr Ala Phe Glu Ala Lys Arg Thr Ile Asn Ala Ile Trp Phe
   65 70 75
   Leu Asn Leu Ala Val Ala Asp Phe Leu Ser Cys Leu Ala Leu Pro
   80 85 90
   Ile Leu Phe Thr Ser Ile Val Gln His His His Trp Pro Phe Gly
   95 100 105
   Gly Ala Ala Cys Ser Ile Leu Pro Ser Leu Ile Leu Leu Asn Met
                   110                 115                 120
   Tyr Ala Ser Ile Leu Leu Leu Ala Thr Ile Ser Ala Asp Arg Phe
                   125                 130                 135
   Leu Leu Val Phe Lys Pro Ile Trp Cys Gln Asn Phe Arg Gly Ala
                   140                 145                 150
   Gly Leu Ala Trp Ile Ala Cys Ala Val Ala Trp Gly Leu Ala Leu
                   155                 160                 165
   Leu Leu Thr Ile Pro Ser Phe Leu Tyr Arg Val Val Arg Glu Glu
                   170                 175                 180
   Tyr Phe Pro Pro Lys Val Leu Cys Gly Val Asp Tyr Ser His Asp
                   185                 190                 195
   Lys Arg Arg Glu Arg Ala Val Ala Ile Val Arg Leu Val Leu Gly
                   200                 205                 210
   Phe Leu Trp Pro Leu Leu Thr Leu Thr Ile Cys Tyr Thr Phe Ile
                   215                 220                 225
   Leu Leu Arg Thr Trp Ser Arg Arg Ala Thr Arg Ser Thr Lys Thr
                   230                 235                 240
   Leu Lys Val Val Val Ala Val Val Ala Ser Phe Phe Ile Phe Trp
                   245                 250                 255
   Leu Pro Tyr Gln Val Thr Gly Ile Met Met Ser Phe Leu Glu Pro
                   260                 265                 270
   Ser Ser Pro Thr Phe Leu Leu Leu Asn Lys Leu Asp Ser Leu Cys
                   275                 280                 285
   Val Ser Phe Ala Tyr Ile Asn Cys Cys Ile Asn Pro Ile Ile Tyr
                   290                 295                 300
   Val Val Ala Gly Gln Gly Phe Gln Gly Arg Leu Arg Lys Ser Leu
                   305                 310                 315
   Pro Ser Leu Leu Arg Asn Val Leu Thr Glu Glu Ser Val Val Arg
                   320                 325                 330
   Glu Ser Lys Ser Phe Thr Arg Ser Thr Val Asp Thr Met Ala Gln
                   335                 340                 345
   Lys Thr Gln Ala Val
                   350
(2) INFORMATION FOR SEQ ID NO:6:
   (i) SEQUENCE CHARACTERISTICS:
      (A) LENGTH: 350 amino acids
      (B) TYPE: amino acid
      (D) TOPOLOGY: linear
   (xi) SEQUENCE DESCRIPTION: SEQ ID NO:6:
   Met Glu Thr Asn Ser Ser Leu Pro Thr Asn Ile Ser Gly Gly Thr
    1              5                   10                  15
   Pro Ala Val Ser Ala Gly Tyr Leu Phe Leu Asp Ile Ile Thr Tyr
                   20                  25                  30
   Leu Val Phe Ala Val Thr Phe Val Leu Gly Val Leu Gly Asn Gly
                   35                  40                  45
   Leu Val Ile Trp Val Ala Gly Phe Arg Met Thr His Thr Val Thr
                   50                  55                  60
   Thr Ile Ser Tyr Leu Asn Leu Ala Val Ala Asp Phe Cys Phe Thr
                   65                  70                  75
   Ser Thr Leu Pro Phe Phe Met Val Arg Lys Ala Met Gly Gly His
                   80                  85                  90
   Trp Pro Phe Gly Trp Phe Leu Cys Lys Phe Val Phe Thr Ile Val
                   95                  100                 105
   Asp Ile Asn Leu Phe Gly Ser Val Phe Leu Ile Ala Leu Ile Ala
                   110                 115                 120
   Leu Asp Arg Cys Val Cys Val Leu His Pro Val Trp Thr Gln Asn
                   125                 130                 135
   His Arg Thr Val Ser Leu Ala Lys Lys Val Ile Ile Gly Pro Trp
                   140                 145                 150
   Val Met Ala Leu Leu Leu Thr Leu Pro Val Ile Ile Arg Val Thr
                   155                 160                 165
   Thr Val Pro Gly Lys Thr Gly Thr Val Ala Cys Thr Phe Asn Phe
                   170                 175                 180
   Ser Pro Trp Thr Asn Asp Pro Lys Glu Arg Ile Asn Val Ala Val
                   185                 190                 195
   Ala Met Leu Thr Val Arg Gly Ile Ile Arg Phe Ile Ile Gly Phe
                   200                 205                 210
   Ser Ala Pro Met Ser Ile Val Ala Val Ser Tyr Gly Leu Ile Ala
                   215                 220                 225
   Thr Lys Ile His Lys Gln Gly Leu Ile Lys Ser Ser Arg Pro Leu
                   230                 235                 240
   Arg Val Leu Ser Phe Val Ala Ala Ala Phe Phe Leu Cys Trp Ser
                   245                 250                 255
   Pro Tyr Gln Val Val Ala Leu Ile Ala Thr Val Arg Ile Arg Glu
                   260                 265                 270
   Leu Leu Gln Gly Met Tyr Lys Glu Ile Gly Ile Ala Val Asp Val
                   275                 280                 285
   Thr Ser Ala Leu Ala Phe Phe Asn Ser Cys Leu Asn Pro Met Leu
                   290                 295                 300
   Tyr Val Phe Met Gly Gln Asp Phe Arg Glu Arg Leu Ile His Ala
                   305                 310                 315
   Leu Pro Ala Ser Leu Glu Arg Ala Leu Thr Glu Asp Ser Thr Gln
                   320                 325                 330
   Thr Ser Asp Thr Ala Thr Asn Ser Thr Leu Pro Ser Ala Glu Val
                   335                 340                 345
   Glu Leu Gln Ala Lys
                   350
(2) INFORMATION FOR SEQ ID NO:7:
   (i) SEQUENCE CHARACTERISTICS:
      (A) LENGTH: 323 amino acids
      (B) TYPE: amino acid
      (D) TOPOLOGY: linear
   (xi) SEQUENCE DESCRIPTION: SEQ ID NO:7:
   Met Thr Pro Thr Thr Thr Thr Ala Glu Leu Thr Thr Glu Phe Asp
   1 5 10 15
   Tyr Asp Glu Asp Ala Thr Pro Cys Val Phe Thr Asp Val Leu Asn
   20 25 30
   Gln Ser Lys Pro Val Thr Leu Phe Leu Tyr Gly Val Val Phe Leu
   35 40 45
   Phe Gly Ser Ile Gly Asn Phe Leu Val Ile Phe Thr Ile Thr Trp
   50 55 60
   Arg Arg Arg Ile Gln Cys Ser Gly Asp Val Tyr Phe Ile Asn Leu
   65 70 75
   Ala Ala Ala Asp Leu Leu Phe Val Cys Thr Leu Pro Leu Trp Met
   80 85 90
   Gln Tyr Leu Leu Asp His Asn Ser Leu Ala Ser Val Pro Cys Thr
   95 100 105
   Leu Leu Thr Ala Cys Phe Tyr Val Ala Met Phe Ala Ser Leu Cys
   110 115 120
   Phe Ile Thr Glu Ile Ala Leu Asp Arg Tyr Tyr Ala Ile Val Tyr
                   125                 130                 135
   Met Arg Tyr Arg Pro Val Lys Gln Ala Cys Leu Phe Ser Ile Phe
                   140                 145                 150
   Trp Trp Ile Phe Ala Val Ile Ile Ala Ile Pro His Phe Met Val
                   155                 160                 165
   Val Thr Lys Lys Asp Asn Gln Cys Met Thr Asp Tyr Asp Tyr Leu
                   170                 175                 180
   Glu Val Ser Tyr Pro Ile Ile Leu Asn Val Glu Leu Met Leu Gly
                   185                 190                 195
   Ala Phe Val Ile Pro Leu Ser Val Ile Ser Tyr Cys Tyr Tyr Arg
                   200                 205                 210
   Ile Ser Arg Ile Val Ala Val Ser Gln Ser Arg His Lys Gly Arg
                   215                 220                 225
   Ile Val Arg Val Leu Ile Ala Val Val Leu Val Phe Ile Ile Phe
                   230                 235                 240
   Trp Leu Pro Tyr His Leu Thr Leu Phe Val Asp Thr Leu Lys Leu
                   245                 250                 255
   Leu Lys Trp Ile Ser Ser Ser Cys Glu Phe Glu Arg Ser Leu Lys
                   260                 265                 270
   Arg Ala Leu Ile Leu Thr Glu Ser Leu Ala Phe Cys His Cys Cys
                   275                 280                 285
   Leu Asn Pro Leu Leu Tyr Val Phe Val Gly Thr Lys Phe Arg Lys
                   290                 295                 300
   Asn Tyr Thr Val Cys Trp Pro Ser Phe Ala Ser Asp Ser Phe Pro
                   305                 310                 315
   Ala Met Tyr Pro Gly Thr Thr Ala
                   320         323
(2) INFORMATION FOR SEQ ID NO:8:
   (i) SEQUENCE CHARACTERISTICS:
      (A) LENGTH: 1495 bases
      (B) TYPE: nucleic acid
      (C) STRANDEDNESS: single
      (D) TOPOLOGY: linear
   (xi) SEQUENCE DESCRIPTION: SEQ ID NO:8:
   ATG GAA ACT CCA AAC ACC ACA GAG GAC TAT GAC ACG 36
   Met Glu Thr Pro Asn Thr Thr Glu Asp Tyr Asp Thr
     1               5                  10
   ACC ACA GAG TTT GAC TAT GGG GAT GCA ACT CCG TGC CAG 75
   Thr Thr Glu Phe Asp Tyr Gly Asp Ala Thr Pro Cys Gln
           15                  20                  25
   AAG GTG AAC GAG AGG GCC TTT GGG GCC CAA CTG CTG CCC 114
   Lys Val Asn Glu Arg Ala Phe Gly Ala Gln Leu Leu Pro
                   30                  35
   CCT CTG TAC TCC TTG GTA TTT GTC ATT GGC CTG GTT GGA 153
   Pro Leu Tyr Ser Leu Val Phe Val Ile Gly Leu Val Gly
       40                  45                  50
   AAC ATC CTG GTG GTC CTG GTC CTT GTG CAA TAC AAG AGG 192
   Asn Ile Leu Val Val Leu Val Leu Val Gln Tyr Lys Arg
               55                  60
   CTA AAA AAC ATG ACC AGC ATC TAC CTC CTG AAC CTG GCC 231
   Leu Lys Asn Met Thr Ser Ile Tyr Leu Leu Asn Leu Ala
   65                  70                  75
   ATT TCT GAC CTG CTC TTC CTG TTC ACG CTT CCC TTC TGG 270
   Ile Ser Asp Leu Leu Phe Leu Phe Thr Leu Pro Phe Trp
           80                  85                  90
   ATC GAC TAC AAG TTG AAG GAT GAC TGG GTT TTT GGT GAT 309
   Ile Asp Tyr Lys Leu Lys Asp Asp Trp Val Phe Gly Asp
                   95                  100
   GCC ATG TGT AAG ATC CTC TCT GGG TTT TAT TAC ACA GGC 348
   Ala Met Cys Lys Ile Leu Ser Gly Phe Tyr Tyr Thr Gly
       105                 110                 115
   TTG TAC AGC GAG ATC TTT TTC ATC ATC CTG CTG ACG ATT 387
   Leu Tyr Ser Glu Ile Phe Phe Ile Ile Leu Leu Thr Ile
               120                 125
   GAC AGG TAC CTG GCC ATC GTC CAC GCC GTG TTT GCC TTG 426
   Asp Arg Tyr Leu Ala Ile Val His Ala Val Phe Ala Leu
   130                 135                 140
   CGG GCA CGG ACC GTC ACT TTT GGT GTC ATC ACC AGC ATC 465
   Arg Ala Arg Thr Val Thr Phe Gly Val Ile Thr Ser Ile
           145                 150                 155
   ATC ATT TGG GCC CTG GCC ATC TTG GCT TCC ATG CCA GGC 504
   Ile Ile Trp Ala Leu Ala Ile Leu Ala Ser Met Pro Gly
                   160                 165
   TTA TAC TTT TCC AAG ACC CAA TGG GAA TTC ACT CAC CAC 543
   Leu Tyr Phe Ser Lys Thr Gln Trp Glu Phe Thr His His
       170                 175                 180
   ACC TGC AGC CTT CAC TTT CCT CAC GAA AGC CTA CGA GAG 582
   Thr Cys Ser Leu His Phe Pro His Glu Ser Leu Arg Glu
               185                 190
   TGG AAG CTG TTT CAG GCT CTG AAA CTG AAC CTC TTT GGG 621
   Trp Lys Leu Phe Gln Ala Leu Lys Leu Asn Leu Phe Gly
   195                 200                 205
   CTG GTA TTG CCT TTG TTG GTC ATG ATC ATC TGC TAC ACA 660
   Leu Val Leu Pro Leu Leu Val Met Ile Ile Cys Tyr Thr
           210                 215                 220
   GGG ATT ATA AAG ATT CTG CTA AGA CGA CCA AAT GAG AAG 699
   Gly Ile Ile Lys Ile Leu Leu Arg Arg Pro Asn Glu Lys
                   225                 230
   AAA TCC AAA GCT GTC CGT TTG ATT TTT GTC ATC ATG ATC 738
   Lys Ser Lys Ala Val Arg Leu Ile Phe Val Ile Met Ile
       235                 240                 245
   ATC TTT TTT CTC TTT TGG ACC CCC TAC AAT TTG ACT ATA 777
   Ile Phe Phe Leu Phe Trp Thr Pro Tyr Asn Leu Thr Ile
               250                 255
   CTT ATT TCT GTT TTC CAA GAC TTC CTG TTC ACC CAT GAG 816
   Leu Ile Ser Val Phe Gln Asp Phe Leu Phe Thr His Glu
   260                 265                 270
   TGT GAG CAG AGC AGA CAT TTG GAC CTG GCT GTG CAA GTG 855
   Cys Glu Gln Ser Arg His Leu Asp Leu Ala Val Gln Val
           275                 280                 285
   ACG GAG GTG ATC GCC TAC ACG CAC TGC TGT GTC AAC CCA 894
   Thr Glu Val Ile Ala Tyr Thr His Cys Cys Val Asn Pro
                   290                 295
   GTG ATC TAC GCC TTC GTT GGT GAG AGG TTC CGG AAG TAC 933
   Val Ile Tyr Ala Phe Val Gly Glu Arg Phe Arg Lys Tyr
       300                 305                 310
   CTG CGG CAG TTG TTC CAC AGG CGT GTG GCT GTG CAC CTG 972
   Leu Arg Gln Leu Phe His Arg Arg Val Ala Val His Leu
               315                 320
   GTT AAA TGG CTC CCC TTC CTC TCC GTG GAC AGG CTG GAG 1011
   Val Lys Trp Leu Pro Phe Leu Ser Val Asp Arg Leu Glu
   325                 330                 335
   AGG GTC AGC TCC ACA TCT CCC TCC ACA GGG GAG CAT GAA 1050
   Arg Val Ser Ser Thr Ser Pro Ser Thr Gly Glu His Glu
           340                 345                 350
   CTC TCT GCT GGG TTC TGACT CAGACCATAG GAGGCCAACC 1090
   Leu Ser Ala Gly Phe
                   355
   CAAAATAAGC AGGCGTGACC TGCCAGGCAC ACTGACCAGC AGCCTGGCTC 1140
   TCCCAGCCAG GTTCTGACTC TTGGCACAGC ATGGAGTCCG CCTCTTGGAT 1190
   AGAGAGGAAT GTAATGGTGG CCTGGGGCTT CTGAGGCTTC TGGGCTTGAG 1240
   TCTTTTCCAT GAACTTCTCC CCTGGTAGAA AAGAAGATGA ATGAGCAAAA 1290
   CCAAATATTC CAGAGACTGG GACTAAGTGT ACCAGAGAAG GGCTTGGACT 1340
   CAAGCAAGAT TTCAGATTTG TGACCATTAG CATTTGTCAA CAAAGTCACC 1390
   CACTTCCCAC TATTGCTTGC ACAAACCAAT TAAACCCAGT AGTGGTGACT 1440
   GTGGGCTCCA TTCAAAGTGA GCTCCTAAGC CATGGGAGAC ACTGATGTAT 1490
   GAGGA 1495
(2) INFORMATION FOR SEQ ID NO:9:
   (i) SEQUENCE CHARACTERISTICS:
      (A) LENGTH: 9 amino acids
      (B) TYPE: amino acid
      (D) TOPOLOGY: linear
   (xi) SEQUENCE DESCRIPTION: SEQ ID NO:9:
   Leu Asn Leu Ala Xaa Ala Asp Xaa Xaa
   1 5 9
(2) INFORMATION FOR SEQ ID NO:10:
   (i) SEQUENCE CHARACTERISTICS:
      (A) LENGTH: 10 amino acids
      (B) TYPE: amino acid
      (D) TOPOLOGY: linear
   (xi) SEQUENCE DESCRIPTION: SEQ ID NO:10:
   Asn Pro Xaa Xaa Tyr Xaa Xaa Xaa Gly Gln
   1 5 10
(2) INFORMATION FOR SEQ ID NO:11:
   (i) SEQUENCE CHARACTERISTICS:
      (A) LENGTH: 9 amino acids
      (B) TYPE: amino acid
      (D) TOPOLOGY: linear
   (xi) SEQUENCE DESCRIPTION: SEQ ID NO:11:
   Asp Arg Tyr Leu Ala Ile Val His Ala
    1               5               9

## Description

This invention relates to the field of cytokine receptors, their antibodies, and their use as diagnostic and therapeutic agents.

### BACKGROUND OF THE INVENTION

Cytokines are biological molecules which affect inflammatory and immune-related effector cells. The inflammatory cytokines, or "chemokines," have a variety of biological properties including selective leukocyte chemotaxis and activation. These chemokines form a superfamily, denoted in the literature alternatively as the PF4 superfamily or the intercrines, that has been divided into two classes based on whether the first two conserved cysteine residues are separated by an intervening amino acid (C-X-C, or α), or whether they are adjacent (C-C, or β). The C-X-C class members include, for example, interleukin-8 (IL-8), melanocyte growth stimulating factor (MGSA), and platelet factor 4 (PF4), while the C-C class includes RANTES (Regulated on Activation, Normal T Expressed and Secreted) and monocyte chemotactic peptide-1 (MCP-1). The C-X-C class exerts proinflammatory activity mainly through their action on neutrophils, whereas the C-C class appears to be monocyte chemoattractants.

Much attention has been.focused on receptor/ligand interactions in this superfamily, with more data being available on C-X-C than C-C chemokine binding. It has become clear that chemokine receptor/ligand interactions on target inflammatory cells seem to be strictly regulated. For example, no cross competition for binding sites has been observed on either monocytes or neutrophils between members of the C-X-C or C-C branches (Leonard, E. J. et al. Immunol. Today 11:97-101, 1990; Samanta, A. K. et al. J. Exp. Med. 169:1185-1189, 1989; Yoshimura, T. et al. J. Immunol. 145:292-7, 1990), consistent with the differential chemoattractant effects on these two cell types. Direct binding data for the C-C chemokines is surprisingly sparse. Human MCP-1 has been reported to bind to monocytes with an affinity of about 2 nM, with no sites detectable on neutrophils (Valente, A. J. et al. Biochem. Biophys. Res. Commun. 176:309-14, 1991; Yoshimura, T. et al. J. Immunol. 145:292-7, 1990). A single report shows human Act-2, a human MIP-1β (HuMIP-1β) variant, binding to between 7, 000 and 45,000 sites on PBMC with an affinity of between 7.8 and 12 nM (Napolitano, M. et al. J. Exp. Med. 172:285-289, 1990). Kwon and colleagues have characterized the binding of murine MIP-1α on a mouse T cell and a macrophage cell line, finding a K_{d} of 1.5 and 0.9 nM, respectively (Oh, K. O. et al. J. Immunol. 147:2978-83, 1991).

Most of the molecular details regarding leukocyte motility remain to be elucidated. Recently, however, the receptors for the anaphylatoxin C5a (Gerard, N. P. et al. Nature 349:614-7, 1991), the bacterial formylated tripeptide fMLP (Boulay, F. et al. Biochemistry 29:11123-11133, 1990), and the C-X-C chemokine IL-8 (Holmes, W. E. et al. Science 253:1278-80, 1991; Murphy, P. M. et al. Science 253:1280-3, 1991) have been cloned using molecular techniques. All of these receptors display amino acid sequences which are predicted to conform to an architecture containing seven-transmembrane-spanning segments connected by a series of intra- and extracellular loops. The primary sequences of these receptors revealed domains which were conserved in receptors associated with cell motility, but not in other seven-transmembrane-spanning receptors.

Accordingly, it is an object of the invention to provide isolated C-C chemokine receptor (C-C CKR-1) for use as a therapeutic or diagnostic reagent.

It is another object of the invention to make variants of C-C CKR-1 for use as antagonists or agonists.

It is another object of the invention to generate antibodies against C-C CKR-1 for use as diagnostic and therapeutic agents.

It is another object of the invention to provide a method for identifying new chemokine receptors.

### SUMMARY OF THE INVENTION

One aspect of the invention is the isolation of the novel chemokine receptor, C-C CKR-1.

In another aspect, the invention provides a composition comprising C-C CKR-1 that is free of contaminating polypeptides of the animal species from which the C-C CKR-1 is derived.

In another aspect of the invention, C-C CKR-1, or fragments thereof (which also may be synthesized by chemical methods), is fused (by recombinant expression or in vitro covalent methods) to an immunogenic polypeptide and this fusion polypeptide, in turn, is used to immunize an animal to raise antibodies against a C-C CKR-1 epitope. Anti-C-C CKR-1 antibodies are recovered from the serum of immunized animals. Alternatively, monoclonal antibodies are prepared from cells of the immunized animal in conventional fashion.

Another aspect of the invention is the use of anti-C-C CKR-1 antibodies in the diagnosis of (in vitro or in vivo) or (when immobilized on an insoluble matrix) the purification of chemokine receptors which bind thereto.

Another aspect of the invention is the derivatization of C-C CKR-1 in vitro to prepare immobilized C-C CKR-1 and labeled C-C CKR-1, particularly for purposes of diagnosis of C-C CKR-1 or its antibodies, or for affinity purification of C-C CKR-1 antibodies themselves.

Another aspect of the invention is the formulation of C-C CKR-1, its derivatives, or its antibodies into physiologically acceptable vehicles, especially for therapeutic use. Such vehicles include sustained-release formulations of C-C CKR-1.

In still other aspects, the invention provides an isolated nucleic acid molecule encoding C-C CKR-1, labeled or unlabeled, and a nucleic acid sequence that is complementary to, or hybridizes under defined conditions to a nucleic acid sequence encoding C-C CKR-1.

In addition, the invention provides a replicable vector comprising the nucleic acid molecule encoding C-C CKR-1 operably linked to control sequences recognized by a host transformed by the vector; host cells transformed with the vector; and a method of using a nucleic acid molecule encoding C-C CKR-1 to effect the production of C-C CKR-1, comprising expressing the nucleic acid molecule in a culture of the transformed host cells and recovering C-C CKR-1 from the host cell culture. The nucleic acid sequence is also useful in hybridization assays for C-C CKR-1 nucleic acid.

Another aspect of the invention is substitutional, deletional, or insertional variants of C-C CKR-1 amino acids and/or glycosyl residues, including variants having non-native glycosylation. These variants are prepared by in vitro or recombinant methods. Sequence variants are optionally screened for immuno-cross-reactivity with C-C CKR-1 and for C-C CKR-1 antagonist or agonist activity.

Another aspect of the invention is a method for identifying new C-C chemokine receptors as set out in the claims below.

Another aspect of the invention is a method for determining the biological activity of a C-C chemokine variant on C-C CKR-1, by transforming a host cell with DNA encoding C-C CKR-1, culturing the host cell to express the receptor on its surface, harvesting the cells, contacting the cells with a C-C chemokine variant, and determining the biological activity of the variant on the receptor.

The invention may be used to provide transgenic animals comprising C-C CKR-1 from another species, animals in which C-C CKR-1 is expressed in a tissue in which it is not ordinarily found, or animals in which C-C CKR-1 is inactivated, by, for example, gene disruption.

### BRIEF DESCRIPTION OF THE FIGURES

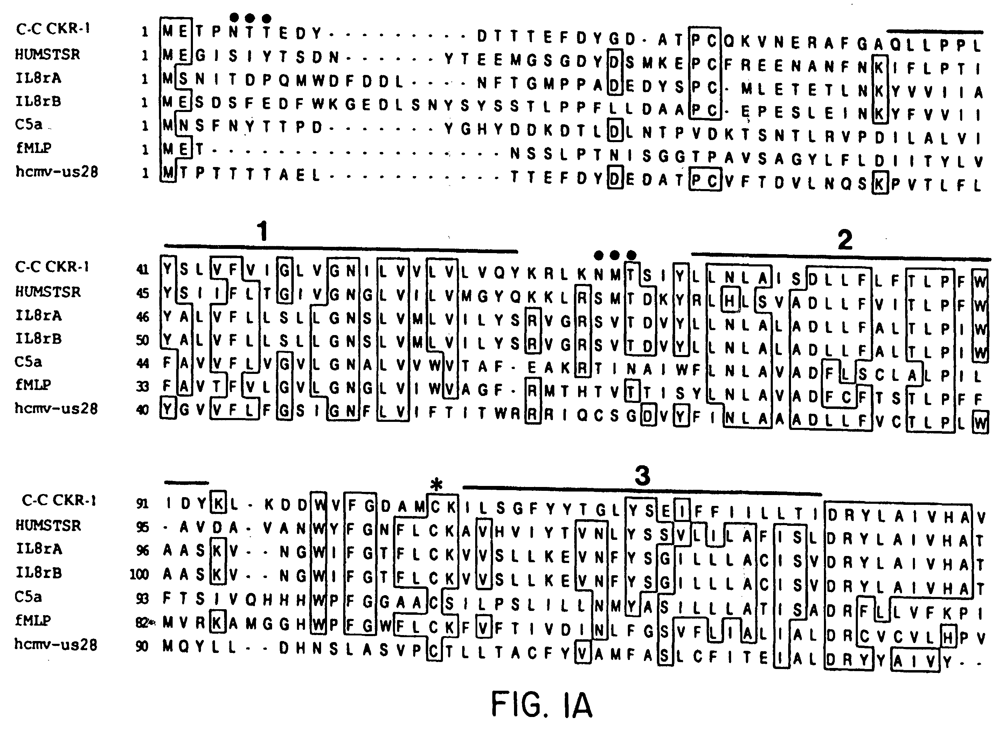

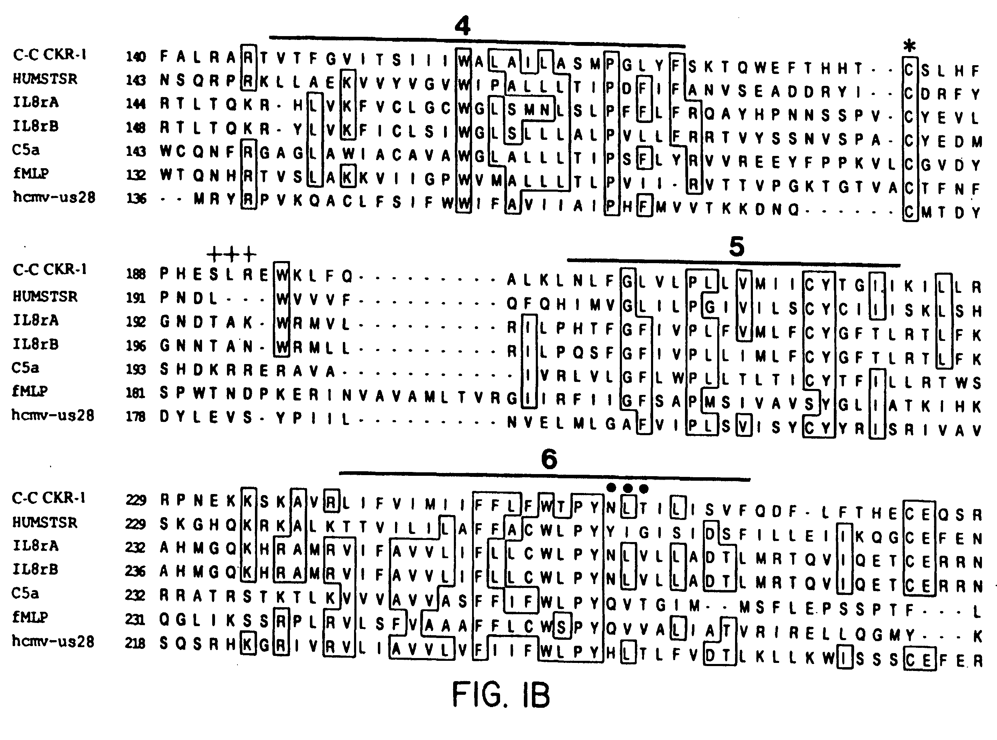

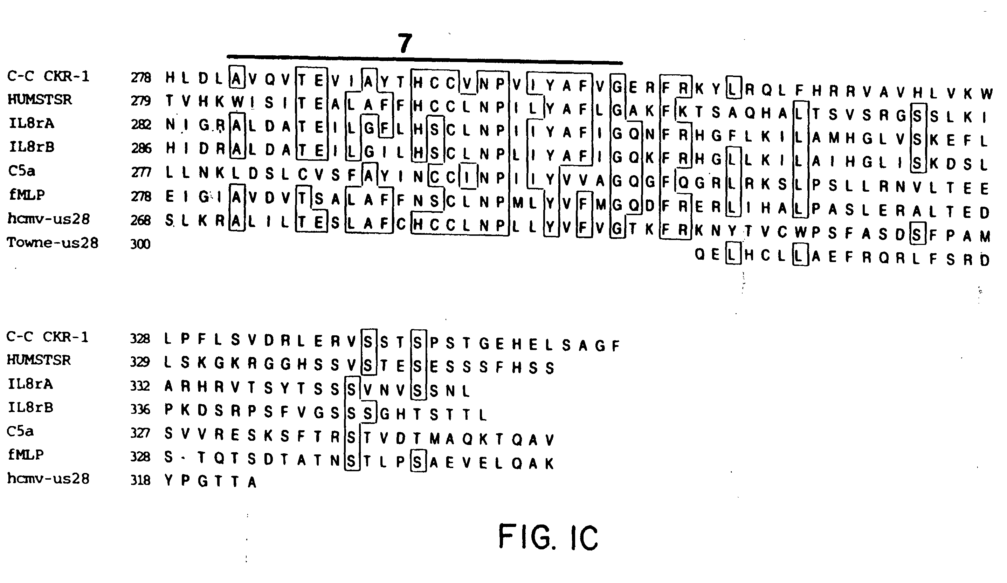

Figure 1 (SEQ ID NOS:1-7) depicts the predicted amino acid sequence of C-C CKR-1, and alignment of the predicted amino acid sequence with HUMSTSR (Genbank accession #M99293), IL8rA (Holmes, W. E. et al. Science 253:1278-80, 1991), IL8rB (Murphy, P. M. et al. Science 253:1280-3, 1991), C5a receptor (Gerard, N. P. et al. Nature 349:614-7, 1991), fMLP receptor (Boulay, F. et al. Biochemistry 29:11123-11133, 1990) and the open reading frame of cytomegalovirus, US28. The seven putative transmembrane spanning domains are.overlined. Glycosylation sites are indicated with black dots above the sequence. The two cysteine residues implicated in disulfide bonding are indicated with asterisks. The consensus site for protein kinase C phosphorylation is indicated as "+". Conserved amino acids appearing more then two times in the alignment are boxed.

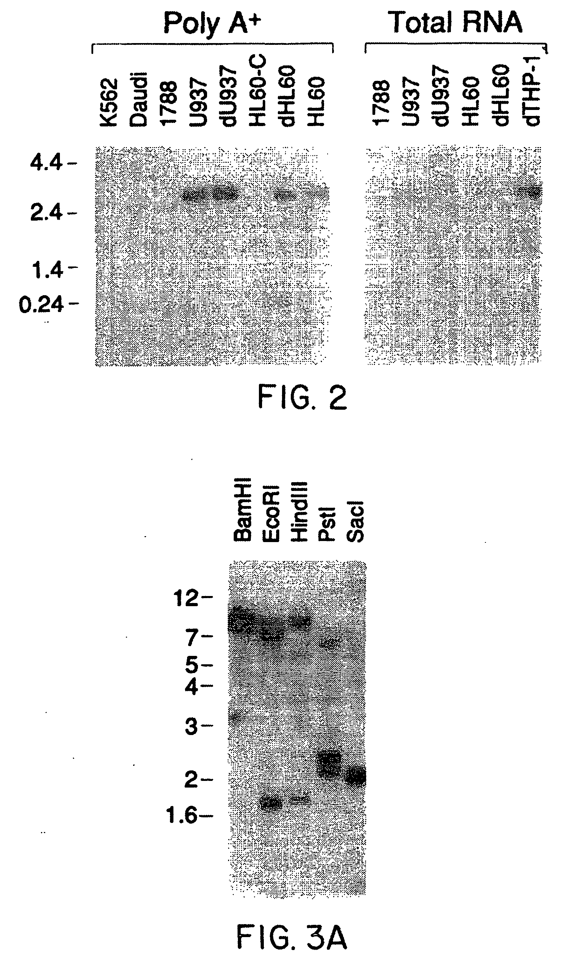

Figure 2 depicts Northern blot analysis of RNA from hematopoietic cells probed with C-C CKR-1. 5 µg of poly A⁺ or 20 µg of total RNA from the cell lines indicated was size fractionated on 1% formaldehyde-agarose, transferred to nitrocellulose and hybridized with radiolabeled C-C CKR-1 cDNA. The filter was washed with 0.5X SSC, 0.1% SDS at 55°C and the autoradiograph was developed after 4-6 hours exposure at -70°C with intensifying screens. RNA molecular weight markers were also run on the gel and are indicated on the side.

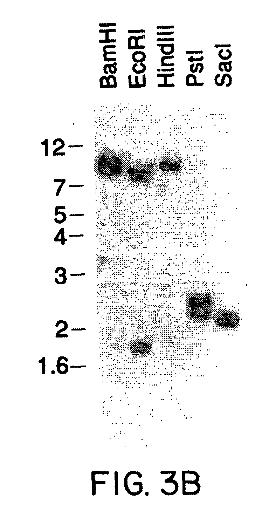

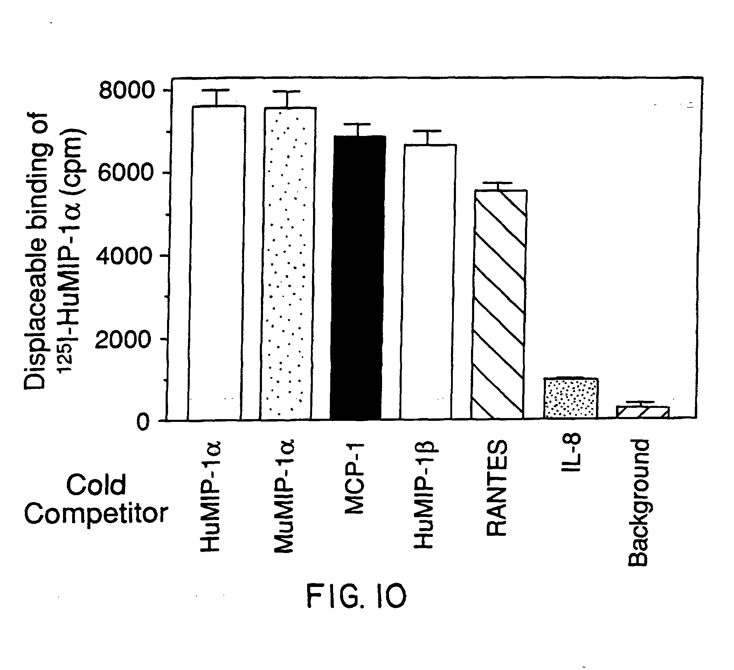

Figures 3A and 3B depict Southern blot analysis of human genomic DNA probed with C-C CKR-1. In Figure 3A, 10 µg of genomic DNA was digested with the restriction enzyme indicated, run on a 0.6% agarose gel, blotted onto Genescreen® and hybridized with radiolabeled C-C CKR-1 cDNA. The filter was washed with 0.5X SSC, 1% SDS at 55°C and the autoradiograph was developed after overnight exposure at -70°C with intensifying screens. DNA molecular weight markers were also run on the gel and are indicated on the side. In Figure 3B, the same blot was washed more stringently with 0.2X SSC, 0.1% SDS at 60°C and autoradiography performed as indicated before.

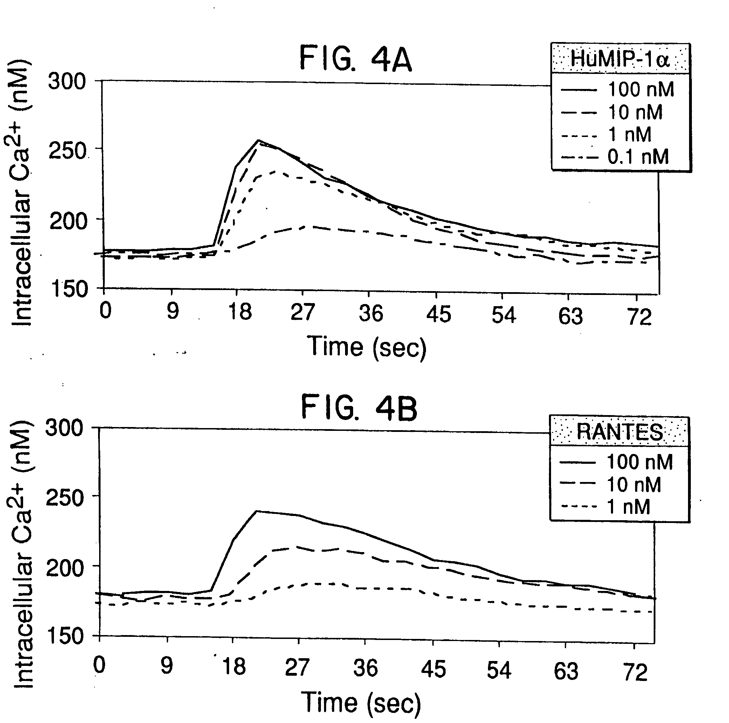

Figures 4A and 4B are graphs depicting intracellular Ca⁺⁺ concentrations of 293 cells transfected with C-C CKR-1 cDNA and challenged with human MIP-1α(HuMIP-1α) and RANTES. In Figure 4A, 50% confluent cells were transfected with 10-20 µg of plasmid DNA by the calcium-phosphate precipitation method. After transient expression for 12-24 hours, cells were harvested, loaded with the calcium probe INDO-1 AM and assayed by spectrofluorometric methods at 37°C with continuous stirring. Various concentrations of HuMIP-1α, as indicated, were added after 12 seconds. The intracellular concentrations of Ca⁺⁺ was determined as described (Naccache, P. H. et al. J. Immunol. 142:2438-44, 1989). In Figure 4B, details were as for Figure 4A, except that various concentration of RANTES, as indicated, were used.

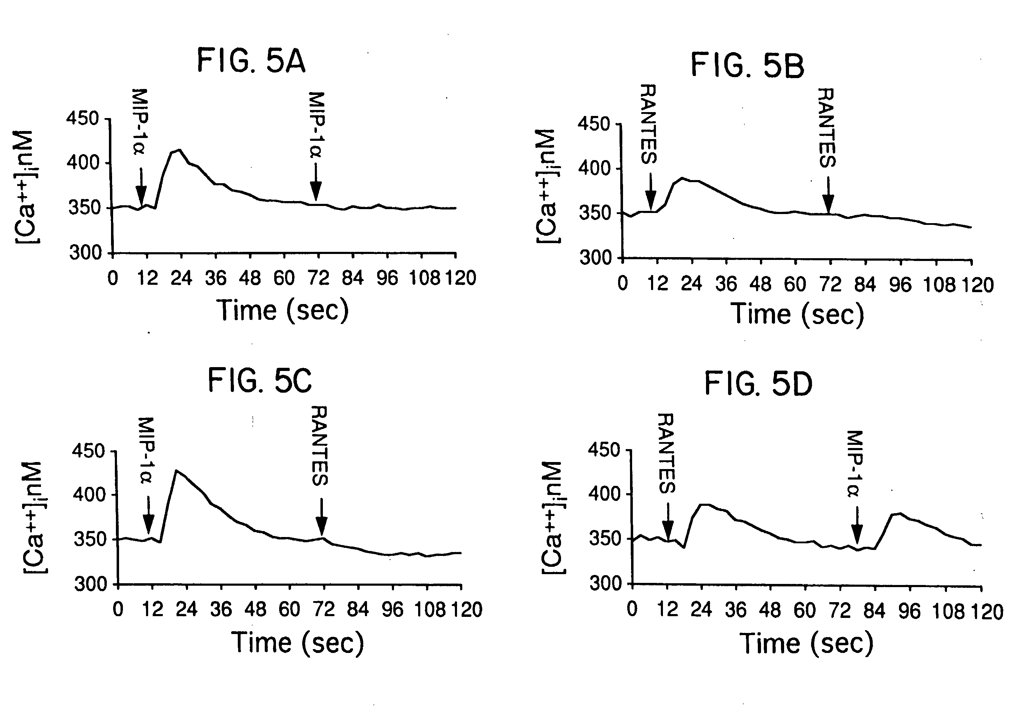

Figures 5A-5D are graphs depicting desensitization in response to the challenge of the same or different ligands by 293 cells transiently expressing C-C CKR-1. Details are as described in Figure 4. The transfected cells were first challenged at 12 seconds with 100 nM of HuMIP-1α or 250 nM of RANTES, and then at 70 seconds with the same concentration of ligands in the order indicated.

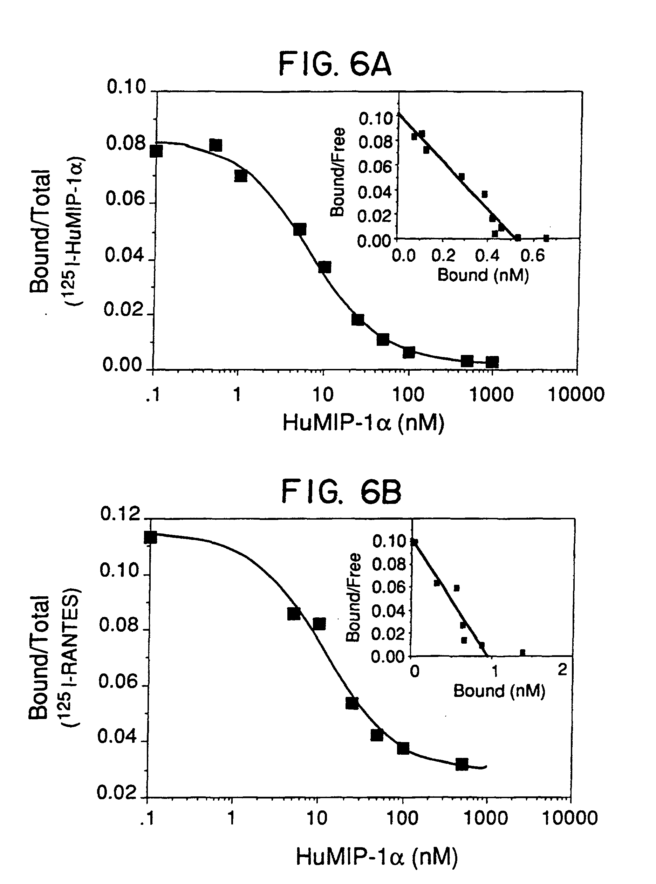

Figures 6A and 6B are graphs depicting the binding of ¹²⁵I-HuMIP-1α and ¹²⁵I-RANTES on 293 cells transfected with C-C CKR-1 cDNA. In Figure 6A, Human embryonic kidney cells (293 cells) were transfected with 10-20 µg plasmid DNA as described in Figure 4. Transfected cells were incubated for 2 hours at 4°C with ¹²⁵I-HuMIP-1α in the presence of increasing concentrations of unlabeled HuMIP-1α. The inset shows Scatchard analysis of the binding data and revealed a K_{d} of 5.1±0.3nM for ¹²⁵I-MIP-1α to C-C CKR-1. Figure 6B depicts displacement of ¹²⁵I-RANTES with unlabeled HuMIP-1α on 293 cells transfected with the C-C CKR-1 cDNA. Scatchard analysis of the binding data revealed a K_{d} of 7.6±1.5nM for the displacement of ¹²⁵I-RANTES to the C-C CKR-1.

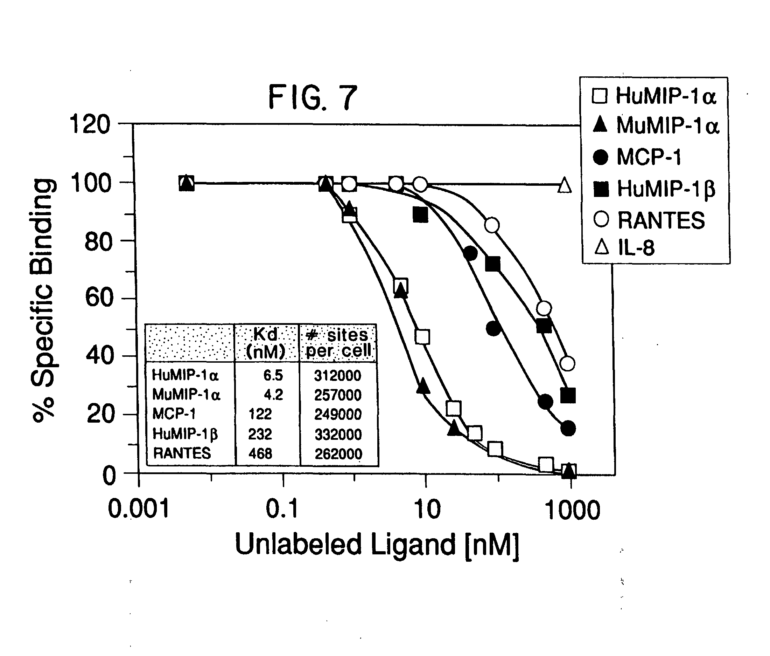

Figure 7 is a graph depicting displacement of ¹²⁵I-HuMIP-1α binding to 293 cells transfected with C-C CKR-1 cDNA. Cells were transfected as outlined in Figure 4 and incubated for 2 hours at 4°C with ¹²⁵I-HuMIP-1α in the presence of increasing concentrations of the cross competing ligands, HuMIP-1α, murine MIP-1α, HuMIP-1β, MCP-1 and IL-8. The K_{d} and the number of sites, shown in the bottom left corner, were determined by Scatchard analysis of the binding data.

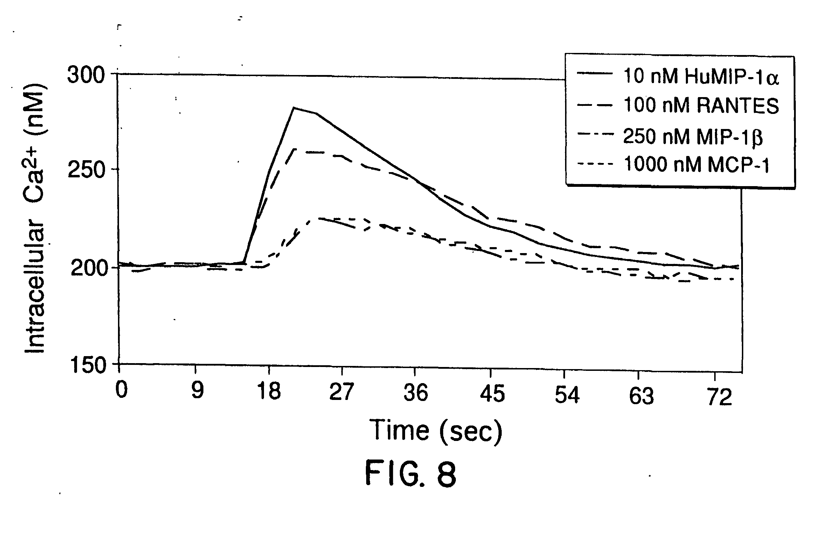

Figure 8 is a graph depicting the intracellular Ca⁺⁺ concentration of 293 cells transiently expressing C-C CKR-1 and challenged with HuMIP-1α, RANTES, HuMIP-1β and MCP-1. Details are as described in Figure 4.

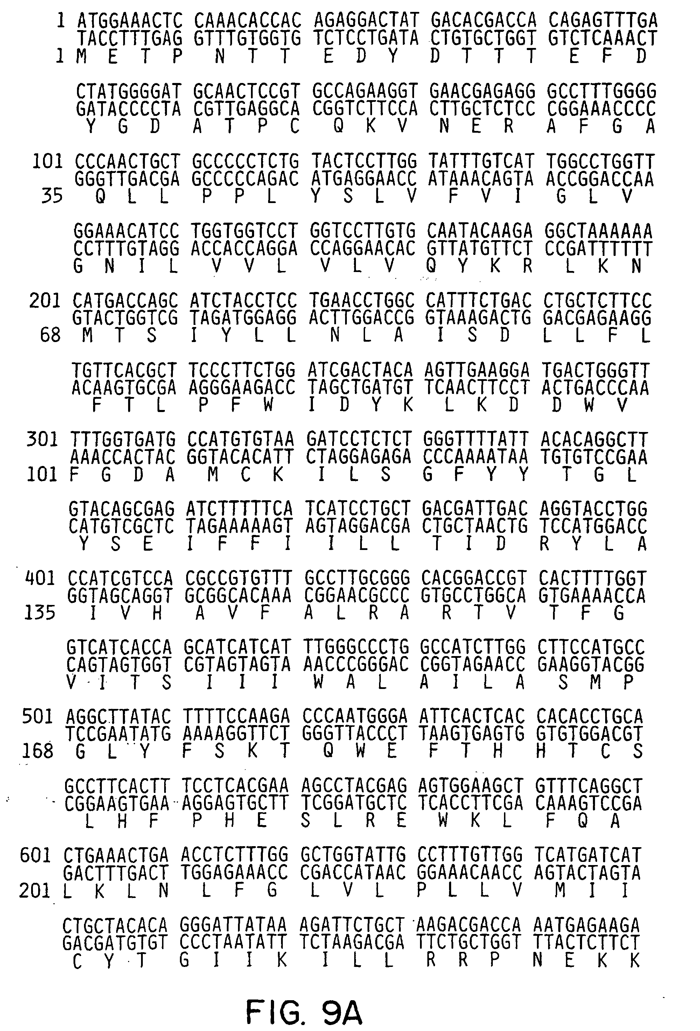

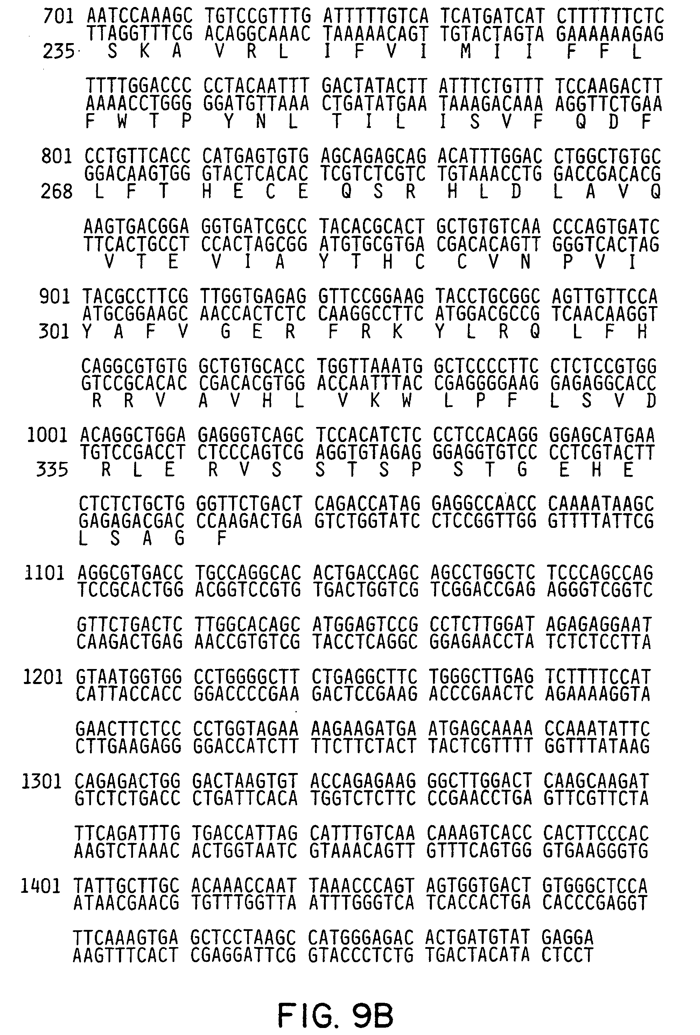

Figure 9 (SEQ ID NO:8) is the nucleotide sequence of C-C CKR-1 and its 3' noncoding region.
Figure 10 is a graph depicting the binding of radiolabeled HuMIP-1α to 293 cells transfected with the coding region of the open reading frame US28 in the cytomegalovirus (CMV) genome. 293 cells were transfected with an expression construct containing the coding sequence of US28 in the sense or antisense orientation. After 12 hours, the cells were harvested and incubated with 0.9 nM of ¹²⁵I-HuMIP-1α in combination with 1 µM of either unlabeled HuMIP-1α, murine MIP-1β, MCP-1, RANTES, or IL-8. The amount of displaceable ¹²⁵I-HuMIP-1α was determined by subtracting the amount of ¹²⁵I-HuMIP-1α bound in the absence of any cold ligand from the amount bound in the presence of cold ligand. Background refers to counts obtained from cells transfected with the antisense orientation of US28.

### DETAILED DESCRIPTION OF THE INVENTION

### A. Definitions

In general, the following words or phrases have the indicated definition when used in the description, examples, and claims.

"C-C CKR-1" is the chemokine receptor described infra together with its amino acid sequence or cellular analogs, alleles, predetermined amino acid sequence mutations, glycosylation variants, and covalent modifications. Embodiments of C-C CKR-1 exclude known chemokine receptors, in particular those which are set forth in the Background section above, and chemokine receptors statutorily obvious from those such chemokine receptors.

"Orphan receptor" is defined as the predicted polypeptide encoded by nucleic acid which hybridizes under low stringency conditions to probes designed from known cytokine receptor nucleic acid sequences or other known sequences likely to have structural similarity to cytokine receptors, or detectable by PCR primers so designed, wherein the predicted polypeptide is not previously known in the art.

"C-C CKR-1 qualitative biological activity" is defined as immunological cross-reactivity with at least one epitope of purified C-C CKR-1.

"Immunologically cross-reactive" is intended to mean that the candidate polypeptide is capable of competitively inhibiting the binding of native C-C CKR-1 to polyclonal antibodies or antisera raised against native C-C CKR-1, respectively.

"Isolated C-C CKR-1 nucleic acid or polypeptide" is a C-C CKR-1 nucleic acid or polypeptide that is identified and separated from at least one contaminant (nucleic acid or polypeptide respectively) with which it is ordinarily associated in nature, such as from the human source of C-C CKR-1 nucleic acid or polypeptide. In preferred embodiments, C-C CKR-1 will be isolated to pharmaceutically acceptable levels of purity with respect to proteins of its species of origin. In preferred embodiments, C-C CKR-1 protein will be purified (1) to greater than 95% by weight of protein, and most preferably more than 99% by weight, (2) to a degree sufficient to obtain at least 15 residues of N-terminal or internal amino acid sequence by an amino acid sequenator commercially available on the filing date hereof, or (3) to homogeneity by conventional nonreducing SDS polyacrylamide gel electrophoresis (SDS-PAGE) using Coomassie blue or, preferably, silver stain. Isolated C-C CKR-1 includes C-C CKR-1 in situ within recombinant cells which do not ordinarily express the C-C CKR-1 in question, since, in this instance, at least one component of C-C CKR-1 natural environment will not be present. Isolated C-C CKR-1 includes C-C CKR-1 in a recombinant cell culture of another species than the species of origin of the C-C CKR-1 since the C-C CKR-1 in such circumstances will be devoid of source polypeptides. Ordinarily, however, isolated C-C CKR-1 will be prepared by at least one purification step.

Isolated C-C CKR-1 nucleic acid includes a nucleic acid that is identified and separated from at least one containment nucleic acid with which it is ordinarily associated in the natural source of the C-C CKR-1 nucleic acid. Isolated C-C CKR-1 nucleic acid thus is present in other than in the form or setting in which it is found in nature. However, isolated C-C CKR-1-encoding nucleic acid includes C-C CKR-1 nucleic acid in ordinarily C-C CKR-1-expressing cells where the nucleic acid is in a chromosomal location different from that of natural cells or is otherwise flanked by a different DNA sequence than that found in nature.

The nucleic acid or polypeptide may be labeled for diagnostic and probe purposes, using a label as described and defined further below in the discussion of diagnostic assays.

"C-C CKR-1 nucleic acid" is defined as RNA or DNA (a) containing at least 25 bases of the genomic or cDNA sequence that encodes C-C CKR-1, (b) is complementary to the genomic or cDNA sequence that encodes C-C CKR-1, (c) which hybridizes to such nucleic acid and remains stably bound to it under stringent conditions, or (d) encodes a polypeptide sharing at least 60%, preferably at least 70%, with the amino acid sequence of C-C CKR-1, and which polypeptide has the ability to bind at least one C-C chemokine. Preferably the hybridizing RNA or DNA contains at least 25 bases, more preferably 40, and more preferably 60 bases which are identical to the sequences encoding the C-C CKR-1 described infra. Optimally, C-C CKR-1 nucleic acid consists essentially only of sequence encoding C-C CKR-1 or the complement of such sequences.

"Stringency" conditions for hybridization are defined by washing conditions after the hybridization reaction. Typically, hybridization conditions are defined as employing overnight incubation at 42°C, in a solution comprising 20% formamide, 5X SSC (150 mM NaCl, 15 mM trisodium citrate), 50 mM sodium phosphate (pH 7.6), 5X Denhardt's solution, 10% dextran sulfate, and 20 µg/ml denatured, sheared salmon sperm DNA. "High stringency" conditions for washing are defined as typically employing 0.2X SSC, 0.1% SDS at 55°C, while "low stringency" conditions for washing are defined as typically employing 0.5X SSC, 1% SDS at 42°C. These conditions are well known in the art. See, for example, Current Protocols in Molecular Biology, eds. Ausubel, et al., Greene Publishing Associates, NY, 1989.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However enhancers do not have to be contiguous. Linking is accomplished by ligation at convenient restriction sites. If such sites do not exist, then synthetic oligonucleotide adapters or linkers are used in accord with conventional practice.

The starting plasmids used to practice this invention are commercially available, are publicly available on an unrestricted basis, or can be constructed from such available plasmids in accord with published procedures. In addition, other equivalent plasmids are known in the art and will be apparent to the ordinary artisan. Methods for restriction enzyme digestion, recovery or isolation of DNA, hybridization analysis, and ligation are conventional and by this time well known to the ordinary artisan. Similarly, the cell lines used to practice this invention are commercially available or are publicly available on an unrestricted basis.

Another method for obtaining the gene of interest is to chemically synthesize it using one of the methods described in Engels et al. (Agnew, Chem. Int. Ed. Engl. 28:716-734, 1989). These methods include triester, phosphite, phosphoramidite and H-phosphonate methods, typically proceeding by oligonucleotide synthesis on solid supports.

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see Sambrook, et al., Molecular Cloning: A Laboratory Manual, Cold Spring Harbor Laboratory Press, Cold Spring Harbor, NY 1989).

Amino acids are referred to by their standard three letter IUPAC abbreviations.

### B. General Methods for Practicing the Invention

### 1. Preparation of Native C-C CKR-1 Nucleic Acid

The use of the singular article "the" with respect to C-C CKR-1 is not intended to suggest that only one DNA sequence encodes C-C CKR-1. In fact, it is expected that alleles, processing intermediates, and predetermined sequence variants described infra will vary in sequence from the DNA encoding native C-C CKR-1. Further, C-C CKR-1 may fall within a subfamily of chemokine receptors having a high degree of sequence homology but which vary sufficiently as to not constitute alleles. All of these sequences fall within the ambit of C-C CKR-1 nucleic acid.

DNA sequences encoding C-C CKR-1 may be either genomic or cDNA. Any representative genomic library may be screened with the probes described below. Methods for genomic DNA preparation and the construction of cDNA libraries are well known in the art. See, for example, Sambrook et al., supra.

### 2. Amino Acid Sequence Variants of C-C CKR-1

Amino acid sequence variants of C-C CKR-1 are prepared by introducing appropriate nucleotide changes into C-C CKR-1 DNA, or by in vitro synthesis of the desired C-C CKR-1 polypeptide. Such variants include, for example, deletions from, or insertions or substitutions of, residues within the amino acid sequence of native C-C CKR-1. Any combination of deletion, insertion, and substitution can be made to arrive at the final construct, provided that the final construct possesses the desired characteristics.

The amino acid changes also may alter post-translational processing of C-C CKR-1, such as changing the number or position of glycosylation sites or by altering its membrane anchoring characteristics.

In designing amino acid sequence variants of C-C CKR-1, the location of the mutation site and the nature of the mutation will depend on C-C CKR-1 characteristic(s) to be modified. The sites for mutation can be modified individually or in series, e.g., by (1) substituting first with conservative amino acid choices and then with more radical selections depending upon the results achieved, (2) deleting the target residue, or (3) inserting residues of the same or a different class adjacent to the located site, or combinations of options 1-3.

A useful method for identification of certain residues or regions of C-C CKR-1 polypeptide that are preferred locations for mutagenesis is called "alanine scanning mutagenesis" as described by Cunningham and Wells (Science 244:1081-1085, 1989). Here, a residue or group of target residues are identified (e.g., charged residues such as arg, asp, his, lys, and glu) and replaced by a neutral or negatively charged amino acid (most preferably alanine or polyalanine) to affect the interaction of the amino acids with the surrounding aqueous environment in or outside the cell. Those domains demonstrating functional sensitivity to the substitutions then are refined by introducing further or other variants at or for the sites of substitution. Thus, while the site for introducing an amino acid sequence variation is predetermined, the nature of the mutation per se need not be predetermined. For example, to optimize the performance of a mutation at a given site, alanine scanning or random mutagenesis may be conducted at the target codon or region and the expressed C-C CKR-1 variants are screened for the optimal combination of desired activity.

C-C CKR-1 variants will exhibit at least a biological activity of the parental sequence, for example, chemokine binding or antigenic activity. Preferably, the antigenically active C-C CKR-1 is a polypeptide that binds to an antibody raised against the polypeptide in its native conformation, "native conformation" generally meaning the polypeptide as found in nature which has not been denatured by chaotropic agents, heat or other treatment that substantially modifies the three dimensional structure of the polypeptide (this can be determined, for example, by migration on nonreducing, nondenaturing sizing gels). Antibody used in determination of antigenic activity is rabbit polyclonal antibody raised by formulating the native non-rabbit polypeptide in Freund's complete adjuvant, subcutaneously injecting the formulation, and boosting the immune response by intraperitoneal injection of the formulation until the titer of anti-polypeptide antibody plateaus.

Amino acid sequence deletions generally range from about 1 to 30 residues, more preferably about 1 to 10 residues, and typically are contiguous. Preferably, deletions are made in regions of the protein that are the least conserved when C-C CKR-1 amino acid sequence is compared with other chemokine receptors. Such deletions will be more likely to modify the biological activity of the polypeptides more significantly than deletions made elsewhere. The number of consecutive deletions will be selected so as to preserve the tertiary structure of C-C CKR-1 in the affected domain, e.g., beta pleated sheet or alpha helix.

Amino acid sequence insertions include amino- and/or carboxyl-terminal fusions ranging in length from one residue to polypeptides containing a hundred or more residues, as well as intrasequence insertions of single or multiple amino acid residues. Intrasequence insertions (i.e., insertions within C-C CKR-1 sequence) may range generally from about 1 to 10 residues, more preferably 1 to 5, most preferably 1 to 3.

Insertional variants of C-C CKR-1 or its extracellular segments include the fusion to the N- or C-terminus of C-C CKR-1 of immunogenic polypeptides, e.g., bacterial polypeptides such as β-lactamase or an enzyme encoded by the E. coli trp locus, or yeast protein, and C-terminal fusions with proteins having a long half-life such as in place of V_{H} or V_{C} domains of immunoglobulins comprising constant regions, albumin, or ferritin (for example, as described in WO 89/02922, published 6 April 1989).

Another group of variants are amino acid substitution variants. These variants have at least one amino acid residue in C-C CKR-1 molecule removed and a different residue inserted in its place. The sites of greatest interest for substitutional mutagenesis include sites identified as the active site(s) of C-C CKR-1, and sites where the amino acids found in C-C CKR-1 from various species are substantially different in terms of side-chain bulk, charge, and/or hydrophobicity.

Other sites of interest are those in which particular residues of C-C CKR-1 are conserved when compared with other chemokine receptors. These positions may be important for the biological activity of C-C CKR-1. These sites, especially those falling within a sequence of at least three other identically conserved sites, are substituted in a relatively conservative manner. Such conservative substitutions are shown in Table I under the heading of preferred substitutions. If such substitutions result in a change in biological activity, then more substantial changes, denominated exemplary substitutions in Table I, or as further described below in reference to amino acid classes, are introduced and the products screened.

**Table I**

| Original Residue | Exemplary Substitutions | Preferred Substitutions |
|---|---|---|
| Ala (A) | ser | ser |
| Arg (R) | lys; gln; asn; ala | lys |
| Asn (N) | gln; his; lys; arg; ala; asp | asp |
| Asp (D) | glu; asn; ala | asn |
| Cys (C) | ser; ala; val | ala |
| Gln (Q) | asn; glu; ala | asn; glu |
| Glu (E) | asp; gln; ala | gln |
| Gly (G) | ala; asn | ala |
| His (H) | asn; gln; lys; arg; ala | asn |
| Ile (I) | leu; val; met; ala; phe | val |
| Leu (L) | ile; val; met; ala; phe | met |
| Lys (K) | arg; gln; asn; met; ala | arg |
| Met (M) | leu; phe; ile;ala | leu |
| Phe (F) | leu; val; ile; ala; tyr | leu |
| Pro (P) | ala | ala |
| Ser (S) | thr; ala | ala |
| Thr (T) | ser; val; ala | ser |
| Trp (W) | tyr; phe; ala | tyr |
| Tyr (Y) | trp; phe; thr; ala; gln | phe |
| Val (V) | ile; leu; met; phe; ala; thr | ala |

Substantial modifications in function or immunological identity of C-C CKR-1 are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another. Such substituted residues may be introduced into regions of C-C CKR-1 that are homologous with other chemokine receptors, or, more preferably, into the non-homologous regions of the molecule.

Any cysteine residues not involved in maintaining the proper conformation of C-C CKR-1 may be substituted, generally with serine, to improve the oxidative stability of the molecule and prevent aberrant cross-linking.

DNA encoding amino acid sequence variants of C-C CKR-1 is prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of C-C CKR-1. These techniques may utilize C-C CKR-1 nucleic acid (DNA or RNA), or nucleic acid complementary to C-C CKR-1 nucleic acid. Oligonucleotide-mediated mutagenesis is a preferred method for preparing substitution, deletion, and insertion variants of C-C CKR-1 DNA. This technique is well known in the art (see, for example, as described by Adelman et al., DNA 2:183, 1983). PCR mutagenesis is also suitable for making amino acid variants of C-C CKR-1 (see Erlich, supra, pp. 61-70). Another method for preparing variants, cassette mutagenesis, is based on the technique described by Wells et al. (Gene 34:315-323, 1985).

### 3. Insertion of DNA into a Cloning Vehicle

The cDNA or genomic DNA encoding native or variant C-C CKR-1 is inserted into a replicable vector for further cloning (amplification of the DNA) or for expression. Many vectors are available, and selection of the appropriate vector will depend on (1) whether it is to be used for DNA amplification or for DNA expression, (2) the size of the DNA to be inserted into the vector, and (3) the host cell to be transformed with the vector. Each vector contains various components depending on its function (amplification of DNA or expression of DNA) and the host cell for which it is compatible. The vector components generally include, but are not limited to, one or more of the following: a signal sequence, an origin of replication, one or more marker genes, an enhancer element, a promoter, and a transcription termination sequence.

### a. Signal Sequence Component

In general, a signal sequence may be a component of the vector, or it may be a part of C-C CKR-1 DNA that is inserted into the vector.

### b. Origin of Replication Component

Both expression.and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Most expression vectors are "shuttle" vectors, i.e. they are capable of replication in at least one class of organisms but can be transfected into another organism for expression. For example, a vector is cloned in E. coli and then the same vector is transfected into yeast or mammalian cells for expression even though it is not capable of replicating independently of the host cell chromosome.

DNA may also be amplified by insertion into the host genome. This is readily accomplished using Bacillus species as hosts, for example, by including in the vector a DNA sequence that is complementary to a sequence found in Bacillus genomic DNA. Transfection of Bacillus with this vector results in homologous recombination with the genome and insertion of C-C CKR-1 DNA. However, the recovery of genomic DNA encoding C-C CKR-1 is more complex than that of an exogenously replicated vector because restriction enzyme digestion is required to excise C-C CKR-1 DNA.

### c. Selection Gene Component

Expression and cloning vectors should contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, e.g. ampicillin, neomycin, methotrexate, or tetracycline, (b) complement auxotrophic deficiencies, or (c) supply critical nutrients not available from complex media, e.g. the gene encoding D-alanine racemase for Bacilli.

One example of a selection scheme utilizes a drug to arrest growth of a host cell. Those cells that are successfully transformed with a heterologous gene express a protein conferring drug resistance and thus survive the selection regimen. Examples of such dominant selection use the drugs neomycin (Southern et al., J. Molec. Appl. Genet. 1:327-341, 1982), mycophenolic acid (Mulligan et al., Science 209:1422-1427, 1980) or hygromycin (Sugden et al., Mol. Cell. Biol. 5:410-413, 1985). The three examples given above employ bacterial genes under eukaryotic control to convey resistance to the appropriate drug G418 or neomycin (geneticin), xgpt (mycophenolic acid), or hygromycin, respectively.

Another example of suitable selectable markers for mammalian cells are those that enable the identification of cells competent to take up C-C CKR-1 nucleic acid, such as dihydrofolate reductase (DHFR) or thymidine kinase. The mammalian cell transformants are placed under selection pressure which only the transformants are uniquely adapted to survive by virtue of having taken up the marker. Selection pressure is imposed by culturing the transformants under conditions in which the concentration of selection agent in the medium is successively changed, thereby leading to amplification of both the selection gene and the DNA that encodes C-C CKR-1. Amplification is the process by which genes in greater demand for the production of a protein critical for growth are reiterated in tandem within the chromosomes of successive generations of recombinant cells. Increased quantities of C-C CKR-1 are synthesized from the amplified DNA.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity, prepared and propagated as described by Urlaub and Chasin, Proc. Natl. Acad. Sci. U.S.A., 77(7):4216-4220, 1980). The transformed cells are then exposed to increased levels of methotrexate. This leads to the synthesis of multiple copies of the DHFR gene, and, concomitantly, multiple copies of other DNA comprising the expression vectors, such as the DNA encoding C-C CKR-1. This amplification technique can be used with any otherwise suitable host, e.g., ATCC No. CCL61 CHO-K1, notwithstanding the presence of endogenous DHFR if, for example, a mutant DHFR gene that is highly resistant to Mtx is employed (EP 117,060). Alternatively, host cells (particularly wild-type hosts that contain endogenous DHFR) transformed or co-transformed with DNA sequences encoding C-C CKR-1, wild-type DHFR protein, and another selectable marker such as aminoglycoside 3' phosphotransferase (APH) can be selected by cell growth in medium containing a selection agent for the selectable marker such as an aminoglycosidic antibiotic, e.g., kanamycin, neomycin, or G418.

A suitable selection gene for use in yeast is the trp1 gene present in the yeast plasmid YRp7 (Stinchcomb et al., Nature 282:39-43, 1979); Kingsman et al., Gene 7:141-152, 1979); or Tschemper et al., Gene 10:157-166, 1980). The trp1 gene provides a selection marker for a mutant strain of yeast lacking the ability to grow in tryptophan, for example, ATCC No. 44076. The presence of the trp1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the Leu2 gene.

### e. Promoter Component

Expression vectors usually contain a promoter that is recognized by the host organism and is operably linked to C-C CKR-1 nucleic acid. Promoters are untranslated sequences located upstream (5') to the start codon of a structural gene (generally within about 100 to 1000 bp) that control the transcription and translation of a particular nucleic acid sequence, such as C-C CKR-1, to which they are operably linked. Such promoters typically fall into two classes, inducible and constitutive. Inducible promoters are promoters that initiate increased levels of transcription from DNA under their control in response to some change in culture conditions, e.g. the presence or absence of a nutrient or a change in temperature. At this time a large number of promoters recognized by a variety of potential host cells are well known. These promoters are operably linked to DNA encoding C-C CKR-1 by removing the promoter from the source DNA by restriction enzyme digestion and inserting the isolated promoter sequence into the vector. Both the native C-C CKR-1 promoter sequence and many heterologous promoters may be used to direct amplification and/or expression of C-C CKR-1 DNA. However, heterologous promoters are preferred, as they generally permit greater transcription and higher yields of expressed C-C CKR-1 as compared to the native C-C CKR-1 promoter.

Promoters suitable for use with prokaryotic hosts include the β-lactamase and lactose promoter systems (Chang et al., Nature 275:617-624, 1978); and Goeddel et al., Nature 281:544-548, 1979), alkaline phosphatase, a tryptophan (trp) promoter system (Goeddel, Nucleic Acids Res. 8(18):4057-4074, 1980) and EP 36,776) and hybrid promoters such as the tac promoter (deBoer et al., Proc. Natl. Acad. Sci. U.S.A. 80:21-25, 1983). However, other known bacterial promoters are suitable. Their nucleotide sequences have been published, thereby enabling a skilled worker to operably ligate them to DNA encoding C-C CKR-1 (Siebenlist et al., Cell 20:269-281, 1980) using linkers or adapters to supply any required restriction sites. Promoters for use in bacterial systems also generally will contain a Shine-Dalgarno (S.D.) sequence operably linked to the DNA encoding C-C CKR-1.

Suitable promoting sequences for use with yeast hosts include the promoters for 3-phosphoglycerate kinase (Hitzeman et al., J. Biol. Chem. 255(24):12073-80, 1980) or other glycolytic enzymes (Hess et al., J. Adv. Enzyme Reg. 7:149-67, 1968); and Holland, Biochemistry 17:4900-4907, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in Hitzeman et al., EP 73,657A. Yeast enhancers also are advantageously used with yeast promoters.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence that may be the signal for addition of the poly A tail to the 3' end of the coding sequence. All of these sequences are suitably inserted into mammalian expression vectors.

C-C CKR-1 transcription from vectors in mammalian host cells is controlled by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis B virus and most preferably Simian Virus 40 (SV40), from heterologous mammalian promoters, e.g. the actin promoter or an immunoglobulin promoter, from heat-shock promoters, and from the promoter normally associated with C-C CKR-1 sequence, provided such promoters are compatible with the host cell systems.

The early and late promoters of the SV40 virus are conveniently obtained as an SV40 restriction fragment that also contains the SV40 viral origin of replication (Fiers et al., Nature 273:113-120, 1978; Mulligan and Berg, Science 209:1422-1427, 1980; Pavlakis et al., Proc. Natl. Acad. Sci. U.S.A. 78:7398-7402, 1981). The immediate early promoter of the human cytomegalovirus is conveniently obtained as a HindIII E restriction fragment (Greenaway et al., Gene 18:355-360, 1982). A system for expressing DNA in mammalian hosts using the bovine papilloma virus as a vector is disclosed in U.S. 4,419,446. A modification of this system is described in U.S. 4,601,978. See also Gray et al., Nature 295:503-508, 1982, on expressing cDNA encoding immune interferon in monkey cells; Reyes et al., Nature 297:598-601, 1982, on expression of human β-interferon cDNA in mouse cells under the control of a thymidine kinase promoter from herpes simplex virus, Canaani and Berg, Proc. Natl. Acad. Sci. U.S.A. 79:5166-5170, 1982, on expression of the human interferon β1 gene in cultured mouse and rabbit cells, and Gorman et al., Proc. Natl. Acad. Sci. U.S.A. 79:6777-6781, 1982, on expression of bacterial CAT sequences in CV-1 monkey kidney cells, chicken embryo fibroblasts, Chinese hamster ovary cells, HeLa cells, and mouse NIH-3T3 cells using the Rous sarcoma virus long terminal repeat as a promoter.

### f. Enhancer Element Component

Transcription of a DNA encoding C-C CKR-1 of this invention by higher eukaryotes is often increased by inserting an enhancer sequence into the vector. Enhancers are cis-acting elements of DNA, usually about from 10-300 bp, that act on a promoter to increase its transcription. Enhancers are relatively orientation and position independent having been found 5' (Laimins et al., Proc. Natl. Acad. Sci. U.S.A. 78:464-8, 1981) and 3' (Lusky et al., Mol. Cell Bio. 3(6):1108-1122, 1983) to the transcription unit, within an intron (Banerji et al., Cell 33:729-740, 1983) as well as within the coding sequence itself (Osborne et al., Mol. Cell Bio. 4(7):1293-1305, 1984). Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18, 1982, on enhancing elements for activation of eukaryotic promoters. The enhancer may be spliced into the vector at a position 5' or 3' to C-C CKR-1 DNA, but is preferably located at a site 5' from the promoter.

### g. Transcription Termination Component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3' untranslated regions of eukaryotic or viral DNAs or cDNAs. These regions contain nucleotide segments transcribed as polyadenylated fragments in the untranslated portion of the mRNA encoding C-C CKR-1. The 3' untranslated regions also include transcription termination sites.

Suitable vectors containing one or more of the above listed components and the desired coding and control sequences are constructed by standard ligation techniques. Isolated plasmids or DNA fragments are cleaved, tailored, and religated in the form desired to generate the plasmids required.

For analysis to confirm correct sequences in plasmids constructed, the ligation mixtures are used to transform E. coli K12 strain 294 (ATCC 31,446) and successful transformants selected by ampicillin or tetracycline resistance where appropriate. Plasmids from the transformants are prepared, analyzed by restriction endonuclease digestion, and/or sequenced by the method of Messing et al., Nucleic Acids Res. 9(2):309-321, 1981, or by the method of Maxam et al., Methods in Enzymology 65:499-560, 1980.

Particularly useful in the practice of this invention are expression vectors that provide for the transient expression in mammalian cells of DNA encoding C-C CKR-1. In general, transient expression involves the use of an expression vector that is able to replicate efficiently in a host cell, such that the host cell accumulates many copies of the expression vector and, in turn, synthesizes high levels of a desired polypeptide encoded by the expression vector. Transient expression systems, comprising a suitable expression vector and a host cell, allow for the convenient positive identification of polypeptides encoded by cloned DNAs, as well as for the rapid screening of such polypeptides for desired biological or physiological properties. Thus, transient expression systems are particularly useful in the invention for purposes of identifying analogs and variants of C-C CKR-1 that have C-C CKR-1-like activity, and for analysis of the effect of the binding of chemokine variants to C-C CKR-1.

Other methods, vectors, and host cells suitable for adaptation to the synthesis of C-C CKR-1 in recombinant vertebrate cell culture are described in Gething et al., Nature 293:620-625, 1981; Mantei et al., Nature 281:40-46, 1979); EP 117,060; and EP 117,058. A particularly useful plasmid for mammalian cell culture expression of C-C CKR-1 is pRK5 (EP pub. no. 307,247) or pSVI6B (U.S. Ser. No. 07/441,574 filed 22 November 1989).

### 4. Selection and Transformation of Host Cells

Suitable host cells for cloning or expressing C-C CKR-1 expression vectors are the prokaryote, yeast, or higher eukaryotic cells described above. Suitable prokaryotes include eubacteria, such as Gram-negative or Gram-positive organisms, for example, E. coli, Bacilli such as B. subtilis, Pseudomonas species such as P. aeruginosa, Salmonella typhimurium, or Serratia marcescens. One preferred E. coli cloning host is E. coli 294 (ATCC 31,446), although other strains such as E. coli B, E. coli χ1776 (ATCC 31,537), and E. coli W3110 (ATCC 27,325) are suitable. These examples are illustrative rather than limiting. Preferably the host cell should secrete minimal amounts of proteolytic enzymes. Alternatively, in vitro methods of cloning, e.g. PCR or other nucleic acid polymerase reactions, are suitable.

In addition to prokaryotes, eukaryotic microbes such as filamentous fungi or yeast are suitable hosts for vectors containing C-C CKR-1 DNA. Saccharomyces cerevisiae, or common baker's yeast, is the most commonly used among lower eukaryotic host microorganisms. However, a number of other genera, species, and strains are commonly available and useful to practice the invention, such as S. pombe (Beach and Nurse, Nature 290:140-143, 1981), Kluyveromyces lactis (Louvencourt et al., J. Bacteriol. 154(2):737-742, 1983), Pichia pastoris (EP 183,070), Trichoderma reesia (EP 244,234), Neurospora crassa (Case et al., Proc. Natl. Acad. Sci. U.S.A. 76:5259-5263, 1979), and Aspergillus hosts such as A. nidulans (Ballance et al., Biochem. Biophys. Res. Commun. 112:284-289, 1983); Tilburn et al., Gene 26:205-221, 1983); Yelton et al., Proc. Natl. Acad. Sci. U.S.A. 81:1470-1474, 1984) and A. niger (Kelly and Hynes, EMBO J. 4:475-479, 1985).

Suitable host cells for the expression of glycosylated C-C CKR-1 polypeptide are derived from multicellular organisms. Such host cells are capable of complex processing and glycosylation activities. In principle, any higher eukaryotic cell culture is workable, whether from vertebrate or invertebrate culture. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruit fly), and Bombyx mori host cells have been identified. See, e.g., Luckow et al., Bio/Technology 6:47-55, 1988); Miller et al., in Genetic Engineering, Setlow, J.K. et al., 8:277-279 (Plenum Publishing, 1986), and Maeda et al., Nature 315:592-594, 1985). A variety of such viral strains are publicly available, e.g., the L-1 variant of Autographa californica NPV and the Bm-5 strain of Bombyx mori NPV, and such viruses may be used as the virus according to the present invention, particularly for transfection of Spodoptera frugiperda cells.

Plant cell cultures of cotton, corn, potato, soybean, petunia, tomato, and tobacco can be utilized as hosts. Typically, plant cells are transfected by incubation with certain strains of the bacterium Agrobacterium tumefaciens, which has been previously manipulated to contain C-C CKR-1 DNA. During incubation of the plant cell culture with A. tumefaciens, the DNA encoding C-C CKR-1 is transferred to the plant cell host such that it is transfected, and will, under appropriate conditions, express C-C CKR-1 DNA. In addition, regulatory and signal sequences compatible with plant cells are available, such as the nopaline synthase promoter and polyadenylation signal sequences. Depicker et al., J. Mol. Appl. Gen. 1: 561-573, 1982). In addition, DNA segments isolated from the upstream region of the T-DNA 780 gene are capable of activating or increasing transcription levels of plant-expressible genes in recombinant DNA-containing plant tissue. See EP 321,196 published 21 June 1989.

However, interest has been greatest in vertebrate cells, and propagation of vertebrate cells in culture (tissue culture) has become a routine procedure in recent years (Tissue Culture, Academic Press, Kruse and Patterson, eds., 1973). Examples of useful mammalian host cell lines are monkey kidney CV1 line transformed by SV40 (COS-7, ATCC CRL 1651); human embryonic kidney line (293 or 293. cells subcloned for growth in suspension culture, Graham et al., J. Gen. Virol. 36:59-72, 1977); baby hamster kidney cells (BHK, ATCC CCL 10); Chinese hamster ovary cells/-DHFR (CHO, Urlaub and Chasin, Proc. Natl. Acad. Sci. U.S.A. 77(7):4216-4220, 1980); mouse sertoli cells (TM4, Mather, Biol. Reprod. 23:243-251, 1980); monkey kidney cells (CV1 ATCC CCL 70); African green monkey kidney cells (VERO-76, ATCC CRL-1587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68, 1982); MRC 5 cells; FS4 cells; and a human hepatoma cell line (Hep G2). Preferred host cells are human embryonic kidney 293 and Chinese hamster ovary cells.

The host chosen for expression may also be a multicellular organism, as in a transgenic animal. Such animals have been produced by transfection of germ cells, somatic cells, or embryos with heterologous DNA, suitably implanting the transfected cells and allowing the cells to mature into or stably integrate into adult animals containing the heterologous DNA. A reproducible percentage of such animals transcribe and express the heterologous DNA as protein which can be identified in tissues including blood or serum. Suitable methods for making transgenic animals are described in U.S. Patent 4,396,601 and Palmiter et al., Nature 300:611-615, 1982.

Host cells are transfected and preferably transformed with the above-described expression or cloning vectors of this invention and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in section 1.82 of Sambrook et al., is generally used for prokaryotes or other cells that contain substantial cell-wall barriers. Infection with Agrobacterium tumefaciens is used for transformation of certain plant cells, as described by Shaw et al., Gene 23:315-330, 1983) and WO 89/05859, published 29 June 1989. For mammalian cells without such cell walls, the calcium phosphate precipitation method described in sections 16.30-16.37 of Sambrook et al., supra, is preferred. General aspects of mammalian cell host system transformations have been described by Axel in U.S. 4,399,216, issued 16 August 1983. Transformations into yeast are typically carried out according to the method of Van Solingen et al., J. Bacteriol. 130(2):946-947, 1977) and Hsiao et al., Proc. Natl. Acad. Sci. U.S.A., 76(8):3829-3833, 1979). However, other methods for introducing DNA into cells such as by nuclear injection, electroporation, or by protoplast fusion may also be used.

### 5. Culturing the Host Cells

Prokaryotic cells used to produce C-C CKR-1 polypeptide of this invention are cultured in suitable media as described generally in Sambrook et al., supra.

The mammalian host cells used to produce C-C CKR-1 of this invention may be cultured in a variety of media. Commercially available media such as Ham's F10 (Sigma), Minimal Essential Medium (MEM, Sigma), RPMI-1640 (Sigma), and Dulbecco's Modified Eagle's Medium (DMEM, Sigma) are suitable for culturing the host cells. In addition, any of the media described in Ham and McKeehan, Meth. Enz. 58:44-93, 1979, Barnes and Sato, Anal. Biochem. 102:255-270, 1980, U.S. 4,767,704; 4,657,866; 4,927,762; or 4,560,655; WO 90/03430; WO 87/00195; U.S. Pat. Re. 30,985; or U.S. 5,122,469 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

The host cells referred to in this disclosure encompass cells in in vitro culture as well as cells that are within a host animal.

### 6. Detecting Gene Amplification/Expression

Gene amplification and/or expression may be measured in a sample directly, for example, by conventional Southern blotting, northern blotting to quantitate the transcription of mRNA (Thomas, Proc. Natl. Acad. Sci. U.S.A. 77:5201-5205, 1980), dot blotting (DNA analysis), or in situ hybridization, using an appropriately labeled probe. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies, which may be labeled with a wide variety of labels, such as radionuclides, fluorescers, enzymes, or the like.

Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of tissue sections and assay of cell culture or body fluids, to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like. A particularly sensitive staining technique suitable for use in the present invention is described by Hsu et al., Am. J. Clin. Path. 75:734-738, 1980.

Antibodies useful for immunohistochemical staining and/or assay of sample fluids may be either monoclonal or polyclonal, and may be prepared in any mammal. Conveniently, the antibodies may be prepared against a native or synthetic C-C CKR-1 polypeptide or variant thereof.

### 7. Purification of C-C CKR-1 Polypeptide

C-C CKR-1 is recovered from cell cultures by solubilizing cell membranes in detergent.

When a human C-C CKR-1 is expressed in a recombinant cell other than one of human origin, C-C CKR-1 is completely free of proteins or polypeptides of human origin. However, it is necessary to purify C-C CKR-1 from recombinant cell proteins or polypeptides to obtain preparations that are substantially homogeneous by protein as to C-C CKR-1. As a first step, the cells are centrifuged to separate them from culture medium, followed by suitable purification procedures such as: fractionation on immunoaffinity or ion-exchange columns; ethanol precipitation; reverse phase HPLC; chromatography on silica or on a cation exchange resin such as DEAE; chromatofocusing; SDS-PAGE; ammonium sulfate precipitation; gel filtration using, for example, Sephadex G-75.

C-C CKR-1 variants in which residues have been deleted, inserted or substituted are recovered in the same fashion as the native C-C CKR-1, taking account of any substantial changes in properties occasioned by the variation. For example, preparation of a C-C CKR-1 fusion with another protein or polypeptide; e.g. a bacterial or viral antigen, facilitates purification; an immunoaffinity column containing antibody to the antigen can be used to adsorb the fusion. Immunoaffinity columns such as a rabbit polyclonal anti-C-C CKR-1 column can be employed to absorb C-C CKR-1 variant by binding it to at least one remaining immune epitope. A protease inhibitor such as phenyl methyl sulfonyl fluoride (PMSF) also may be useful to inhibit proteolytic degradation during purification, and antibiotics may be included to prevent the growth of adventitious contaminants. One skilled in the art will appreciate that purification methods suitable for native C-C CKR-1 may require modification to account for changes in the character of C-C CKR-1 or its variants upon expression in recombinant cell culture.

### 8. Covalent Modifications of C-C CKR-1 Polypeptides

Covalent modifications of C-C CKR-1 polypeptide or its glycosyl substituents are included within the scope of this invention. Both native C-C CKR-1 and amino acid sequence variants of C-C CKR-1 may be covalently modified. Covalent modifications of C-C CKR-1, fragments thereof or antibodies thereto are introduced into the molecule by reacting targeted amino acid residues of C-C CKR-1, fragments thereof, or C-C CKR-1 antibody with an organic derivatizing agent that is capable of reacting with selected side chains or the N- or C-terminal residues. Most commonly, C-C CKR-1 and its antibodies are covalently bonded to detectable groups used in diagnosis, e.g. enzymes, radio isotopes, spin labels, antigens, fluorescent or chemiluminescent groups and the like.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidazole)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method described above being suitable.

Carboxyl side groups (aspartyl or glutamyl) are selectively modified by reaction with carbodiimides (R'-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for cross-linking C-C CKR-1, its fragments or antibodies to a water-insoluble support matrix or surface for use in methods for purifying anti-C-C CKR-1 antibodies, and vice versa. Commonly used cross-linking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis-(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming cross-links in the presence of light. Alternatively, reactive water-insoluble matrices such as cyanogen bromide-activated carbohydrates and the reactive substrates described in U.S. 3,969,287; 3,691,016; 4,195,128; 4,247,642; 4,229,537; and 4,330,440 are employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains (T.E. Creighton, Proteins: Structure and Molecular Properties, W.H. Freeman & Co., San Francisco, pp. 79-86 1983), acetylation of the N-terminal amine, and amidation of any C-terminal carboxyl group.

Another type of covalent modification of C-C CKR-1 polypeptide included within the scope of this invention comprises altering the native glycosylation pattern of the polypeptide. By altering is meant deleting one or more carbohydrate moieties found in the native polypeptide, and/or adding one or more glycosylation sites that are not present in the native polypeptide.

Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

Addition of glycosylation sites to C-C CKR-1 polypeptide is conveniently accomplished by altering the amino acid sequence such that it contains one or more of the above-described tri-peptide sequences (for N-linked glycosylation sites). The alteration may also be made by the addition of, or substitution by, one or more serine or threonine residues to the native C-C CKR-1 sequence (for O-linked glycosylation sites). For ease, C-C CKR-1 amino acid sequence is preferably altered through changes at the DNA level, particularly by mutating the DNA encoding C-C CKR-1 polypeptide at preselected bases such that codons are generated that will translate into the desired amino acids. The DNA mutation(s) may be made using methods described above under the heading of "Amino Acid Sequence Variants of C-C CKR-1 Polypeptide".

Another means of increasing the number of carbohydrate moieties on C-C CKR-1 polypeptide is by chemical or enzymatic coupling of glycosides to the polypeptide. These procedures are advantageous in that they do not require production of the polypeptide in a host cell that has glycosylation capabilities for N- and O- linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine; or hydroxyproline, (e) aromatic residues such as those of phenylalanine, tyrosine, or tryptophan, or (f) the amide group of glutamine. These methods are described in WO 87/05330 published 11 September 1987, and in Aplin and Wriston (CRC Crit. Rev. Biochem. pp. 259-306, 1981).

Removal of carbohydrate moieties present on the native C-C CKR-1 polypeptide may be accomplished chemically or enzymatically. Chemical deglycosylation requires exposure of the polypeptide to the compound trifluoromethanesulfonic acid, or an equivalent compound. This treatment results in the cleavage of most or all sugars except the linking sugar (N-acetylglucosamine or N-acetylgalactosamine), while leaving the polypeptide intact. Chemical deglycosylation is described by Hakimuddin et al. (Arch. Biochem. Biophys. 259:52-57, 1987) and by Edge et al. (Anal. Biochem. 118:131-137, 1981). Enzymatic cleavage of carbohydrate moieties on polypeptides can be achieved by the use of a variety of endo- and exo- glycosidases as described by Thotakura et al. (Meth. Enzymol. 138:350-359, 1987).

Glycosylation at potential glycosylation sites may be prevented by the use of the compound tunicamycin as described by Duskin et al. (J. Biol. Chem. 257:3105-3109, 1982). Tunicamycin blocks the formation of protein-N-glycoside linkages.

The C-C CKR-1 also may be entrapped in microcapsules prepared, for example, by coacervation techniques or by interfacial polymerization (for example, hydroxymethylcellulose or gelatin-microcapsules and poly-[methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Osol, A., ed., 1980).

C-C CKR-1 preparations are also useful in generating antibodies, for use as standards in assays for C-C CKR-1 (e.g. by labeling C-C CKR-1 for use as a standard in a radioimmunoassay, enzyme-linked immunoassay, or radioreceptor assay), in affinity purification techniques, and in competitive-type receptor binding assays when labeled with radioiodine, enzymes, fluorophores, spin labels, and the like.

Since it is often difficult to predict in advance the characteristics of a variant C-C CKR-1, it will be appreciated that some screening of the recovered variant will be needed to select the optimal variant. For example, a change in the immunological character of C-C CKR-1 molecule, such as affinity for a given antibody, is measured by a competitive-type immunoassay. The variant is assayed for changes in the suppression or enhancement of its activity by comparison to the activity observed for native C-C CKR-1 in the same assay. Other potential modifications of protein or polypeptide properties such as redox or thermal stability, hydrophobicity, susceptibility to proteolytic degradation, or the tendency to aggregate with carriers or into multimers are assayed by methods well known in the art.

### 9. Therapeutic Compositions and Administration of C-C CKR-1

Therapeutic formulations of C-C CKR-1 (including its C-C CKR-1 binding fragments) or antibodies thereto are prepared for storage by mixing C-C CKR-1 having the desired degree of purity with optional physiologically acceptable carriers, excipients, or stabilizers (Remington's Pharmaceutical Sciences, supra), in the form of lyophilized cake or aqueous solutions. Acceptable carriers, excipients or stabilizers are nontoxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics, or polyethylene glycol (PEG).

The C-C CKR-1 or antibody to be used for in vivo administration must be sterile. This is readily accomplished by filtration through sterile filtration membranes, prior to or following lyophilization and reconstitution. The C-C CKR-1 ordinarily will be stored in lyophilized form or in solution.

Therapeutic C-C CKR-1 or antibody compositions generally are placed into a container having a sterile access port, for example, an intravenous solution bag or vial having a stopper pierceable by a hypodermic injection needle.

The route of C-C CKR-1 or antibody administration is in accord with known methods, e.g. injection or infusion by intravenous, intraperitoneal, intracerebral, intramuscular, intraocular, intraarterial, or intralesional routes, or by sustained release systems as noted below.

Suitable examples of sustained-release preparations include semipermeable polymer matrices in the form of shaped articles, e.g. films, or microcapsules. Sustained release matrices include polyesters, hydrogels, polylactides (U.S. 3,773,919, EP 58,481), copolymers of L-glutamic acid and y ethyl-L-glutamate (Sidman et al., Biopolymers 22:547-556, 1983), poly (2-hydroxyethylmethacrylate) (Langer et al., J. Biomed. Mater. Res. 15:167-277, 1981; Langer, Chem. Tech., 12:98-105, 1982), ethylene vinyl acetate (Langer et al., supra) or poly-D-(-)-3-hydroxybutyric acid (EP 133,988). Sustained-release C-C CKR-1 or antibody compositions also include liposomally entrapped C-C CKR-1 or antibody. Liposomes containing C-C CKR-1 or antibody are prepared by methods known per se: DE 3,218,121; Epstein et al., Proc. Natl. Acad. Sci. U.S.A. 82:3688-3692, 1985; Hwang et al., Proc. Natl. Acad. Sci. U.S.A. 77:4030-4034, 1980; EP 52,322; EP 36,676; EP 88,046; EP 143,949; EP 142,641; Japanese patent application 83-118008; U.S. 4,485,045 and 4,544,545; and EP 102,324. Ordinarily the,liposomes are of the small (about 200-800 Angstroms) unilamelar type in which the lipid content is greater than about 30 mol. % cholesterol, the selected proportion being adjusted for the optimal C-C CKR-1 or antibody therapy.

An effective amount of C-C CKR-1 or antibody to be employed therapeutically will depend, for example, upon the therapeutic objectives, the route of administration, and the condition of the patient. For example, it is expected that C-C CKR-1 will be therapeutically effective in the treatment of cytokine-mediated inflammation. Accordingly, it will be necessary for the therapist to titer the dosage and modify the route of administration as required to obtain the optimal therapeutic effect. Typically, the clinician will administer C-C CKR-1 or antibody until a dosage is reached that achieves the desired effect. The progress of this therapy is easily monitored by conventional assays.

### 10. C-C CKR-1 Antibody Preparation

Polyclonal antibodies to C-C CKR-1 generally are raised in animals by multiple subcutaneous (sc) or intraperitoneal (ip) injections of C-C CKR-1 and an adjuvant. Immunization with recombinant cells transformed with C-C CKR-1 (e.g. mouse or CHO cells transformed with human C-C CKR-1) may be satisfactory, or it may be useful to separate C-C CKR-1 and conjugate it or a fragment containing the target amino acid sequence to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N = C = NR, where R and R¹ are different alkyl groups.

Animals ordinarily are immunized against the cells or immunogenic conjugates or derivatives by combining 1 mg or 1 µg of C-C CKR-1 in Freund's complete adjuvant and injecting the solution intradermally at multiple sites. One month later the animals are boosted with 1/5 to 1/10 the original amount of conjugate in Freund's complete adjuvant by subcutaneous injection at multiple sites. 7 to 14 days later animals are bled and the serum is assayed for anti-C-C CKR-1 titer. Animals are boosted until the titer plateaus. Preferably, the animal is boosted with the conjugate of the same C-C CKR-1, but conjugated to a different protein and/or through a different cross-linking agent. Conjugates also can be made in recombinant cell culture as protein fusions. Also, aggregating agents such as alum are used to enhance the immune response.

Another option is to employ combinatorial variable domain libraries and screening methods to identify the desired anti-C-C CKR-1 antibodies.

Monoclonal antibodies are prepared by recovering spleen cells from immunized animals and immortalizing the cells in conventional fashion, e.g. by fusion with myeloma cells or by Epstein-Barr (EB)-virus transformation and screening for clones expressing the desired antibody.

The monoclonal antibody preferably is specific for each target C-C CKR-1 polypeptide. The antibody is selected to be either agonistic, antagonistic or to have no effect on the activity of or binding of the C-C CKR-1.

### 11. Uses of C-C CKR-1 Nucleic Acid, and Antibodies

The nucleic acid encoding C-C CKR-1 may be used as a diagnostic for tissue specific typing. For example, such procedures as in situ hybridization, and northern and Southern blotting, and PCR analysis may be used to determine whether DNA and/or RNA encoding C-C CKR-1 are present in the cell type(s) being evaluated.

Isolated C-C CKR-1 polypeptide may be used in quantitative diagnostic assays as a standard or control against which samples e.g., from erythrocytes, containing unknown quantities of C-C CKR-1 may be compared. Recombinant cells which express C-C CKR-1 can be used in assays for C-C CKR-1 ligands in the same fashion as for example neutrophils are used in IL-8 assays. The C-C CKR-1 polypeptides, fragments or cells (as such, or derivatized) also can be used as immunogens in the production of antibodies to C-C CKR-1, or for the purification of such antibodies from ascites or recombinant cell culture media.

C-C CKR-1 antibodies are useful in diagnostic assays for C-C CKR-1 expression in specific cells or tissues wherein the antibodies are labeled in the same fashion as C-C CKR-1 described above and/or are immobilized on an insoluble matrix. C-C CKR-1 antibodies also are useful for the affinity purification of C-C CKR-1 from recombinant cell culture or natural sources. The C-C CKR-1 antibodies that do not detectably cross-react with other chemokine receptors can be used to purify each C-C CKR-1 free from other homologous chemokine receptors. C-C CKR-1 antibodies that are PF4 superfamily antagonists are useful as antiinflammatory agents or in the treatment of other PF4 superfamily-mediated disorders.

Suitable diagnostic assays for C-C CKR-1 and its antibodies are well known per se. Such assays include competitive and sandwich assays, and steric inhibition assays. Competitive and sandwich methods employ a phase-separation step as an integral part of the method while steric inhibition assays are conducted in a single reaction mixture. Fundamentally, the same procedures are used for the assay of C-C CKR-1 and for substances that bind C-C CKR-1, although certain methods will be favored depending upon the molecular weight of the substance being assayed. Therefore, the substance to be tested is referred to as an analyte, irrespective of its status otherwise as an antigen or antibody, and proteins that bind to the analyte are denominated binding partners, whether they be antibodies, cell surface receptors, or antigens.

Analytical methods for C-C CKR-1 or its antibodies all use one or more of the following reagents: labeled analyte analog, immobilized analyte analog, labeled binding partner, immobilized binding partner and steric conjugates. The labeled reagents also are known as "tracers."

The label used (and this is also useful to label C-C CKR-1 nucleic acid for use as a probe) is any detectable functionality that does not interfere with the binding of analyte and its binding partner. Numerous labels are known for use in immunoassay, examples including moieties that may be detected directly, such as fluorochrome, chemiluminescent, and radioactive labels, as well as moieties, such as enzymes, that must be reacted or derivatized to be detected. Examples of such labels include the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Pat. No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

Conventional methods are available to bind these labels covalently to proteins or polypeptides. For instance, coupling agents such as dialdehydes, carbodiimides, dimaleimides, bis-imidates, bis-diazotized benzidine, and the like may be used to tag the antibodies with the above-described fluorescent, chemiluminescent, and enzyme labels. See, for example, U.S. Pat. Nos. 3,940,475 (fluorimetry) and 3,645,090 (enzymes); Hunter et al., Nature 194:495-496, 1962; David et al., Biochemistry 13:1014-1021, 1974; Pain et al., J. Immunol. Methods 40:219-230, 1981; and Nygren, J. Histochem. and Cytochem. 30:407-412, 1982. Preferred labels are enzymes such as horseradish peroxidase and alkaline phosphatase.

The conjugation of such label, including the enzymes, to the antibody is a standard manipulative procedure for one of ordinary skill in immunoassay techniques. See, for example, O'Sullivan et al., "Methods for the Preparation of Enzyme-antibody Conjugates for Use in Enzyme Immunoassay," in Methods in Enzymology, ed. J.J. Langone and H. Van Vunakis, Vol. 73 (Academic Press, New York, New York, 1981), pp. 147-166. Such bonding methods are suitable for use with C-C CKR-1 or its antibodies, all of which are proteinaceous.

Immobilization of reagents is required for certain assay methods. Immobilization entails separating the binding partner from any analyte that remains free in solution. This conventionally is accomplished by either insolubilizing the binding partner or analyte analog before the assay procedure, as by adsorption to a water-insoluble matrix or surface (Bennich et al., U.S. 3,720,760), by covalent coupling (for example, using glutaraldehyde cross-linking), or by insolubilizing the partner or analog afterward, e.g., by immunoprecipitation.

Other assay methods, known as competitive or sandwich assays, are well established and widely used in the commercial diagnostics industry.

Competitive assays rely on the ability of a tracer analog to compete with the test sample analyte for a limited number of binding sites on a common binding partner. The binding partner generally is insolubilized before or after the competition and then the tracer and analyte bound to the binding partner are separated from the unbound tracer and analyte. This separation is accomplished by decanting (where the binding partner was preinsolubilized) or by centrifuging (where the binding partner was precipitated after the competitive reaction). The amount of test sample analyte is inversely proportional to the amount of bound tracer as measured by the amount of marker substance. Dose-response curves with known amounts of analyte are prepared and compared with the test results to quantitatively determine the amount of analyte present in the test sample. These assays are called ELISA systems when enzymes are used as the detectable markers.

Another species of competitive assay, called a "homogeneous" assay, does not require a phase separation. Here, a conjugate of an enzyme with the analyte is prepared and used such that when anti-analyte binds to the analyte the presence of the anti-analyte modifies the enzyme activity. In this case, C-C CKR-1 or its immunologically active fragments are conjugated with a bifunctional organic bridge to an enzyme such as peroxidase. Conjugates are selected for use with anti-C-C CKR-1 so that binding of the anti-C-C CKR-1 inhibits or potentiates the enzyme activity of the label. This method per se is widely practiced under the name of EMIT.

Steric conjugates are used in steric hindrance methods for homogeneous assay. These conjugates are synthesized by covalently linking a low-molecular-weight hapten to a small analyte so that antibody to hapten substantially is unable to bind the conjugate at the same time as anti-analyte. Under this assay procedure the analyte present in the test sample will bind anti-analyte, thereby allowing anti-hapten to bind the conjugate, resulting in a change in the character of the conjugate hapten, e.g., a change in fluorescence when the hapten is a fluorophore.

Sandwich assays particularly are useful for the determination of C-C CKR-1 or C-C CKR-1 antibodies. In sequential sandwich assays an immobilized binding partner is used to adsorb test sample analyte, the test sample is removed as by washing, the bound analyte is used to adsorb labeled binding partner, and bound material is then separated from residual tracer. The amount of bound tracer is directly proportional to test sample analyte. In "simultaneous" sandwich assays the test sample is not separated before adding the labeled binding partner. A sequential sandwich assay using an anti-C-C CKR-1 monoclonal antibody as one antibody and a polyclonal anti-C-C CKR-1 antibody as the other is useful in testing samples for C-C CKR-1 activity.

The foregoing are merely exemplary diagnostic assays for C-C CKR-1 and antibodies. Other methods now or hereafter developed for the determination of these analytes are included within the scope hereof, including the bioassays described above.

The following examples are offered by way of illustration and not by way of limitation.

### C. EXPERIMENTAL EXAMPLES

### Example I

### Isolation of Orphan Receptors

An "orphan receptor" cloning strategy was employed in an attempt to isolate cDNAs encoding C-C chemokine receptors. The cell surface receptors for the chemoattractants C5a (Gerard, N. P. et al. Nature 349:614-7, 1991), the bacterial tripeptide fMLP (Boulay, F. et al. Biochemistry 29:11123-11133, 1990), as well as two receptors for the C-X-C chemokine IL-8 receptors A and B (IL8rA and IL8rB) have been recently cloned (Holmes, W. E. et al. Science 253:1278-80, 1991; Murphy, P. M. et al. Science 253:1280-3, 1991) and found to belong to the superfamily of receptor proteins whose structures are predicted to transverse the cell membrane seven times (Dohlman, H. G. et al. Annu. Rev. Biochem. 60:653-88, 1991). Since seven-transmembrane-spanning molecules are typically linked to G-proteins whose function can be inhibited by pertussis toxin, we assumed that the receptors for the C-C chemokine class of proteins would also share the seven transmembrane architecture. Accordingly, two degenerate oligonucleotides corresponding to conserved amino acid sequences in two transmembrane regions (TM) of the IL8rA, the C5a and the fMLP receptors were synthesized. The first oligonucleotide corresponded to a region in TM2: LNLA(L/V)AD(L/F) (L/G) (SEQ ID NO:9) and the second in TM7: NP(I/M)(I/L)Y(A/V)(F/V)(I/M/A)GQ (SEQ ID NO:10).

These oligomers were then used as primers in RT-PCR experiments using cDNA substrates from different hematopoietic cell types known to respond to C-C chemokines including peripheral blood mononuclear cells (PBMC), and the cell lines U937, HL60 and THP-1. PCR was performed as follows. 1-2 µg of total RNA from different hematopoietic cell lines were used as substrates in RT-PCR (Larrick, J. W. Trends Biotech. 10:146-152, 1992), as recommended by the supplier (Perkin Elmer, Norwalk, CT). Degenerate oligonucleotides corresponding to conserved regions of chemoattractant receptors were used in the PCR. PCR conditions were as follows: 94°C for 0.5', 50-55°C for 0.5' 72°C for 0.5-1', 30 cycles. PCR products were blunt-end cloned into the SmaI site of pBS (Stratagene. LaJolla, CA) as previously described (Nguyen, T. et al. Gene 109:211-8, 1991). Plasmid DNA was isolated using the Quiagen kit (Quiagen Inc., Chatsworth, CA) as recommended by the supplier. Sequencing was performed with the Sequenase kit (USBC, Cleveland, OH) as recommended by the supplier.

Subcloning and sequencing of the PCR products revealed the presence of IL8rA, IL8rB, C5a receptor and two novel clones having characteristics of seven-transmembrane-segment receptors and marked similarity to the two IL-8 receptors. Partial functional characterization of these orphan receptors revealed that they do not bind to HuMIP-1α. However, it was noted that these two clones, which were more related to the IL-8 receptors than to other seven-transmembrane-spanning receptors, possessed a new conserved amino acid motif at the end of TM3, DRYLAIVHA (SEQ ID NO:11), which seemed to define a subfamily of IL-8 receptor-related seven-transmembrane-spanning molecules that excluded the C5a and the fMLP receptors. Therefore, a second round of RT-PCR/orphan cloning was carried out using the TM2 degenerate oligonucleotide and a DRYLAIVHA (SEQ ID NO:11) degenerate oligonucleotide. In addition, to increase the chances of obtaining C-C chemokine receptors, cDNA was obtained from cultured human monocytes, which bind radiolabeled HuMIP-1α and from B Cells, which respond chemotactically to HuMIP-1α and used in the PCR reaction. Cloning and sequencing of these PCR products revealed several additional unique seven-transmembrane-spanning receptors.

Monocytes were cultured by standard techniques commonly known in the art. In summary, buffy coat cells were separated on a Ficoll gradient. Mononuclear cells recovered from the interface were repeatedly centrifuged to remove platelets. The monocytes were separated from other mononuclear cells by adhering them to tissue culture dishes. The non-adherent cells were washed off and the adherent monocytes were then were cultured for 48-72 hours before use.

Alternatively, genomic or cDNA could be screened for the presence of orphan receptor genes by traditional Southern blot hybridization to the oligomers described above, or to probes designed from cloned DNA of a seven-transmembrane spanning protein.

### Example II

### Characterization of C-C CKR-1 DNA

A cDNA corresponding to one clone, JOSH1, was isolated by screening a λgt10 cDNA library made from PMA (phorbol 12-myristate 13-acetate) treated HL60 cells with ³²P-labeled restriction fragments. Phage DNA was isolated and the inserts of the λgt10 clones were obtained by PCR, employing 20 cycles with primers flanking the insert and the enzyme Pfu DNA polymerase (Stratagene, LaJolla, CA), as recommend by the supplier. The PCR products were subcloned into the SmaI site of pRK5 as described above.

The nucleotide sequence of JOSH1 revealed an open reading frame of 1065 bases, encoding a protein of 355 amino acids (Figures 1 and 9). The deduced amino acid sequence, provisionally designated as the C-C chemokine receptor 1 (C-C CKR-1), has key features related to G-protein-linked receptors of the seven-transmembrane-spanning receptor superfamily. For example, it has seven hydrophobic regions predicted to span the cell membrane, and cysteine residues in the first and the second extracellular loops that are implicated in forming a disulfide bond (Figure 1). However, certain features of the predicted C-C CKR-1 protein make it distinct from classical seven-transmembrane-spanning receptors. The carboxyl-terminus is relatively short and lacks cysteine residues involved in membrane anchorage via a palmitoylated moiety (O'Dowd, B. F. et al. J. Biol. Chem. 264:7564-9, 1989) and the segments between the transmembrane domains are relatively short, a feature consistent in other chemoattractant receptors (Boulay, F. et al. Biochemistry 30:2993-9, 1991; Boulay, F. et al. Biochemistry 29:11123-11133, 1990; Gerard, N. P. et al. Nature 349:614-7, 1991; Holmes, W. E. et al. Science 253:1278-80, 1991; Murphy, P. M. et al. Science 253:1280-3, 1991). There are three potential glycosylation sites in the C-C CKR-1 (Figure 1), one in the N-terminus, one in the first cytoplasmic loop and the third in TM6. This latter site is unlikely to be glycosylated since it is predicted to be embedded in the cell membrane. Finally, there is a consensus sequence for a protein kinase C phosphorylation site, at position 192, but this position is predicted to be extracellular.

The deduced amino acid sequence of C-C CKR-1 was compared to other G-protein linked chemoattractant receptors (Figure 1). The IL8rA and IL8rB showed about 32% sequence identity to C-C CKR-1, while the C5a and fMLP receptors showed about 23% identity (Figure 1). A putative seven transmembrane spanning molecule, HUMSTSR, which has been recently been deposited in Genbank (accession #M99293) by Federsspiel, et al., was found to have about 31% identity with C-C CKR-1. The sequence of this molecule is identical to one of the putative receptors isolated in our first attempt at RT-PCR cloning described above. The ligand for HUMSTSR has not yet been identified. The second group of receptors which are as closely related to the C-C CKR-1 are the neuropeptide Y and the angiotensin II receptors (data not shown). Lastly, an open reading frame in the cytomegalovirus genome, designated US28, has about 33% identity with the C-C CKR-1 and about 60% identity in the N-terminal region before TM1 with C-C CKR-1.

### Example III

### Northern and Southern analysis of C-C CKR-1

The expression of the C-C CKR-1 was assessed in a limited panel of hematopoietic cell lines using Northern blot analysis. Northern blot hybridization was performed as follows. Total RNA was isolated using the guanidinium isothiocyanate-CsCl procedure (Sambrook et al., 1989) or by the RNAzol method as recommended by the supplier. Poly A⁺ RNA was isolated using Dynabeads oligodT (DYNAL, Great Neck, NY) as recommended by the supplier. HL60 mRNA designated "HL60-C" in this report was obtained from Clontech (Palo Alto, CA). 20 µg of total RNA or 5 µg of poly A+ RNA was fractionated on formaldehyde-agarose gels, blotted to a nitrocellulose membrane and hybridized with the C-C CKR-1 cDNA (Korneluk, R. G. et al. J. Biol. Chem. 261:8407-8413, 1986).

A single band of about 3 kb was detected in pre-monocytic cell lines, e.g. undifferentiated or differentiated U937 and HL60 cells (Figure 2), but not in a commercially available preparation of HL60 RNA (HL60-C, Figure 2). Lower levels of mRNA were also detected in B cell lines 1788 and Daudi, but little or no RNA was detected in K562 cells (Figure 2). The highest levels were detected in PMA-treated THP-1 cells, where a strong signal was obtained when 20 mg of total RNA was analyzed (Figure 2).

Southern blot hybridization was performed as follows. Human genomic DNA, obtained from Clontech (Palo Alto, CA), was restriction digested, blotted onto Genescreen® (Dupont) and hybridized (Neote, K., et al. J. Clin. Invest. 86:1524-31, 1990) with the C-C CKR-1 cDNA. The hybridization pattern indicated that the C-C CKR-1 gene could be intronless. Furthermore, it suggested the existence of a second related gene or possibly a pseudogene. Figures 3A and 3B represent low and high stringency washes of the same blot of human genomic DNA which has been digested with several restriction enzymes. Under high stringency conditions (Figure 3B), single restriction fragments hybridizing to the C-C CKR-1 cDNA were detected when genomic DNA was digested with BamHI, HindIII or SacI and, as predicted from the cDNA sequence, two fragments are detected in DNA digested with EcoRI and PstI. However, low stringency hybridization revealed the presence of additional bands that hybridized to the C-C CKR-1 cDNA e.g. an approximately 7 kb PstI fragment and an approximately 1.6 kb HindIII fragment were detected (Figure 3A). These bands disappeared when the blots were washed under high stringency conditions while the other bands remained unchanged between the two washes.

### Example IV

### Signaling through the C-C CKR-1 in response to HuMIP-1α and RANTES

MCP-1, RANTES, HuMIP-1α and HuMIP-1β induce a rapid and transient increase in intracellular Ca⁺⁺ in human monocytes (Rollins, B. J. et al. Blood 78:1112-6, 1991; Sozzani, S. et al. J. Immunol. 147:2215-21, 1991). To determine if C-C CKR-1 was a functional C-C chemokine receptor, it was transiently expressed in human kidney 293 cells and intracellular Ca⁺⁺ levels in response to different C-C chemokines were measured.

Recombinant RANTES was expressed in E. coli and purified as described by Kuna et al.(J. Immunol. 149:636-42, 1992b). HuMIP-1α and HuMIP-1β was expressed in E. coli, and purified as described by Rot et al. (J. Exp. Med., in press). HuMIP-1α was iodinated by the chloroglycoluril method (Fraker et al. Biochem. Biophys. Res. Comm. 80:849-857, 1978) to an initial specific activity of 472 Ci/mmol. The labeled HuMIP-1a was purified by a combination of gel filtration and reversed-phase HPLC. The ¹²⁵I-labeled RANTES, specific activity 2200 Ci/mmol, was obtained from Dupont/NEN (Boston, MASS). Recombinant MCP-1 and murine MIP-1α were obtained from Peprotech (Rocky Hill, NJ).

Human embryonic kidney 293 cells were transfected with 10-20 µg of plasmid DNA by the calcium-phosphate method as described (Schall, T. J. et al. Eur. J. Immunol. 22:1477-81, 1992) or the modified calcium phosphate method (Chen, C. et al. Mol. Cell. Biol. 7:2745-2752, 1987). The transfected cells were assayed after transient expression for 12-24 hours.

Intracellular Ca⁺⁺ measurements were done on the SLM 8000C essentially as described (Naccache, P. H. et al. J. Immunol. 142:2438-44, 1989), with minor modifications: IND0-1-AM, was used at 20 mg/ml final concentration and 2-4 X10⁶ cells were used per assay.

A 100 nM dose of RANTES, HuMIP-1α, HuMIP-1β and MCP-1 was initially used as a first approximation of the maximum physiologically-relevant concentration. When transfected cells, loaded with the calcium probe INDO-1-AM, were challenged with 100 nM of either HuMIP-1α or RANTES, a rapid increase in intracellular Ca⁺⁺ was observed (Figure 4). Challenge with the same dose of MCP-1 or HuMIP-1β produced little detectable Ca⁺⁺ flux (data not shown). Control cells transfected with vector alone or vector containing C-C CKR-1 in the opposite (non-coding) orientation did not respond to any of the ligands (data not shown). The signaling responses were dose dependent, 10 nM of HuMIP-1α and 100 nM of RANTES were sufficient to give a maximal or near maximal response (Figure 4A and 4B).

Rapid, successive exposure to the same ligand is known to desensitize the signaling capacity of G-protein linked receptors (Schild, H. O. (1973), "Receptor classification with special reference to β-adrenergic receptors," In Drug Receptors, H.P. Rang, ed. (University Press), pp. 29-36). In addition, desensitization can also occur when the two different agonists signal through the same receptor. HuMIP-1α clearly blocks the ability of the C-C CKR-1 receptor to transmit a second Ca⁺⁺ signal when HuMIP-1α is added to transfected cells twice in succession (Figure 5A). Similarly, RANTES blocks the response to a second challenge at the same concentration (Figure 5B). When HuMIP-1α is added first, it blocks the response to RANTES, indicating that complete desensitization has occurred (Figure 5C). However, challenging C-C CKR-1 cDNA transfected cells with 250 nM RANTES did not prevent a subsequent Ca⁺⁺ flux by the addition of 100 nM HuMIP-1α (Figure 5D), indicating that receptor desensitization had not occurred. However, the subsequent Ca⁺⁺ flux by HuMIP-1α is reduced, from a intracellular Ca⁺⁺ change of about 70 nM (Figure 5A and C) to about 40 nM (Figure 5D), suggesting that a partial desensitization has occurred.

### Example V

### Binding of HuMIP-1α and RANTES to C-C cKR-1

In order to further investigate the interaction of HuMIP-1α and RANTES to the cloned C-C CKR-1, direct binding experiments with ¹²⁵I-labeled ligands were carried out.

Binding assays were performed as described previously (Horuk, R. et al. J. Biol. Chem. 262:16275-16278, 1987). Transfected cells (2 x 10⁶ cells per ml) were incubated with radiolabeled ligands and varying concentrations of unlabeled ligands at 4 °C for 2 hours. The incubation was terminated by removing aliquots from the cell suspension and separating cells from buffer by centrifugation through a silicon/paraffin oil mixture as described previously (Robb, R. J. et al. J. Exp. Med. 160:1126-1146, 1984). Non-specific binding was determined in the presence of 1 µM unlabeled ligand. Individual assay determinations, representative of at least three separate experiments were plotted. The binding data were curve fit with the computer program LIGAND (Munson, P. J. et al. Anal. Biochem. 107:220-239, 1980) modified for the IBM PC (McPherson, G. A. Comp. Prog. Biomed. 17:107-114, 1983) to determine the affinity (K_{d}), number of sites, and nonspecific binding. The curves shown are the binding isotherms determined by LIGAND.

Thus, when 293 cells transiently expressing C-C CKR-1 were incubated with ¹²⁵I-HuMIP-1α and increasing concentrations of unlabeled HuMIP-1α, displaceable binding of ¹²⁵I-HuMIP-1α to the C-C CKR-1 was observed (Figure 6A). Scatchard analysis showed a dissociation constant (K_{d}) of 5.1±0.3nM and about 130000 sites/cell. This K_{d} is within the range of that determined for HuMIP-1α binding to human monocytes and suggests that C-C CKR-1 is at least one of the HuMIP-1α receptors present on monocytes. Direct binding of RANTES to the C-C CKR-1 however could not be accomplished, i.e., ¹²⁵I-RANTES could not be displaced by unlabeled RANTES. Interestingly, as the amount of unlabeled RANTES was increased in the binding assay, a concomitant increase of ¹²⁵I-RANTES bound to cells was observed (data not shown). Since the C-C CKR-1 transduces a signal (mobilizes Ca⁺⁺) in response to RANTES (Figure 4), and therefore, the ligand must be binding to the cloned receptor, the reason for the unusual binding profile observed for ¹²⁵I-RANTES is not clear. Similar binding phenomenon are obtained if other target cells responding to RANTES are used. Interestingly, ¹²⁵I-RANTES could be displaced by unlabeled HuMIP-1α on 293 cells transiently expressing C-C CKR-1 (Figure 6B). Scatchard analysis of this heterologous displacement suggested a K_{d} of 7.6±1.5nM (about 350000 sites/cell) and is consistent with the K_{d} of HuMIP-1α binding data described above. These observations suggested to us initially that the C-C CKR-1 binds to HuMIP-1α and RANTES and subsequently transduces a signal by increasing the intracellular Ca⁺⁺ levels

### Example VI

### Displacement of HuMIP-1α by Heterologous Chemokines

To further characterize the binding properties of the C-C CKR-1, and in particular to attempt to define a K_{d} for RANTES binding to the cloned C-C CKR-1, heterologous displacement of ¹²⁵I-HuMIP-1α was done with RANTES and also HuMIP-1β, MCP-1, IL-8, and murine MIP-1α. Interestingly, all C-C chemokines (RANTES, MIP-lb and MCP-1) displaced ¹²⁵I-HuMIP-1α, but the C-X-C chemokine IL-8 did not (Figure 7). The K_{d} for RANTES, HuMIP-1b and MCP-1, and murine MIP-1α as determined from Scatchard analysis is 468±280, 232±70.1, 122±39.3, and 4.2±2.7 nM respectively, and in each case about 250000-350000 sites/cell are present (Figure 7). These results revealed a broad ligand specificity of the C-C CKR-1, including some lack of species specificity in binding. More importantly, it suggested that the binding affinity between the ligand and the receptor does not predict the signaling efficacy resulting from that interaction, i.e. although HuMIP-1β and MCP-1 bind with a higher affinity then does RANTES, 100 nM of MIP-1β or MCP-1 do not transmit a signal via the cloned receptor, whereas RANTES does (Figure 4).

### Example VII

### Calcium Mobilization in Response to HuMIP-1β and MCP-1

To determine the relevance of HuMIP-1β and MCP-1 binding to C-C CKR-1, C-C CKR-1 cDNA transfected 293 cells were challenged with high doses of HuMIP-1β and MCP-1 and intracellular Ca⁺⁺ levels were determined. A concentration of 1 mM of the recombinant MCP-1 had to be used to produce a Ca⁺⁺ flux. This Ca⁺⁺ flux, however, was only about 20% of the maximum response obtained by HuMIP-1α or RANTES (Figure 8). Lower concentrations gave an almost undetectable flux (data not shown). Control experiments utilizing THP-1 cells and 50-100 nM of MCP-1 gave the expected strong Ca⁺⁺ fluxes (a transient increase of 200-300 nM Ca⁺⁺, data not shown). Similarly, 250 nM of HuMIP-1β was needed to produce a Ca⁺⁺ flux in 293 cells transiently expressing C-C CKR-1 that was about 20% of the maximal signal obtained by HuMIP-1α or RANTES (Figure 8). These results support the suggestion that the binding affinities HuMIP-1β and MCP-1 do not reflect their signaling capabilities.

### Example VIII

### Binding of HuMIP-1α to 293 Cells Expressing the Protein Encoded by US28

Since the amino acid sequence of C-C CKR-1 is about 35% identical to an open reading frame in the human CMV genome designated as US28, we sought to determine if the protein encoded by this open reading frame would bind HuMIP-1α. The US28 open reading frame was amplified from a stock of Towne strain cytomegalovirus (a kind gift of Dr. Philip Dormitzer) with primers flanking the coding region using PCR and the thermostable DNA polymerase Pfu. The PCR product was subcloned into the expression vector pRK5 and its identity confirmed by sequencing. Plasmid DNA containing US28 in the sense orientation and the opposite, antisense orientation was transfected into 293 cells. Transfected cells were then used to determine binding of radiolabeled HuMIP-1α. 293 cells transfected with the US28 coding sequence in the sense orientation bound ¹²⁵I-HuMIP-1α, whereas negligible binding was obtained in cells transfected with the antisense orientation (Figure 10). The radiolabeled HuMIP-1α could also be displaced with 1 µM of murine MIP-1α, HuMIP-1β, RANTES, and MCP-1, but not by the C-X-C chemokine, IL-8 (Figure 10). These results indicate that the protein encoded by US28 specifically binds C-C chemokines and not C-X-C chemokines.

## Claims

1. An isolated C-C CKR-1 polypeptide which has
(i) the ability to bind at least one C-C chemokine, and
(ii) the amino acid sequence depicted in Figure 1 or a sequence which shares at least 60% sequence identity over its entire length with the sequence of Figure 1.

2. A polypeptide as claimed in claim 1 which has an amino acid sequence which shares at least 70% sequence identity over its entire length with the sequence of Figure 1.

3. A polypeptide as claimed in claim 2 which has the amino acid sequence depicted in Figure 1.

4. A polypeptide as claimed in any one of the preceding claims which has the ability to bind a C-C chemokine selected from: RANTES, HuMIP-1α, HuMIP-1β or MCP-1.

5. A composition comprising the C-C CKR-1 polypeptide of claim 1, wherein the C-C CKR-1 polypeptide is derived from a natural source, and which composition is free of a contaminating polypeptide of the animal species that is the source of the C-C CKR-1 polypeptide.

6. An antibody that is capable of binding the C-C CKR-1 polypeptide of claim 1 that does not cross-react with another chemokine receptor.

7. An antibody as claimed in claim 6 which is a monoclonal antibody.

8. An isolated nucleic acid molecule encoding the C-C CKR-1 polypeptide of claim 1.

9. The nucleic acid molecule of claim 8 which is DNA and contains greater than about 25 bases.

10. The nucleic acid molecule of claim 8 which is cDNA.

11. The nucleic acid molecule of claim 8 which is a genomic sequence.

12. The nucleic acid molecule of claim 8 further comprising a promoter operably linked to the nucleic acid sequence.

13. The nucleic acid molecule of claim 12, wherein the promoter is heterologous to the C-C CKR-1 polypeptide.

14. An expression vector comprising the nucleic acid sequence of claim 8 operably linked to control sequences recognized by a host cell transformed with the vector.

15. A host cell transformed with the vector of claim 14.

16. A method of determining the presence of a C-C CKR-1 nucleic acid, comprising hybridizing nucleic acid according to claim 8 or its complement to a test sample nucleic acid and determining the presence of C-C CKR-1 nucleic acid which hybridizes thereto.

17. A method of amplifying a nucleic acid test sample comprising priming a nucleic acid polymerase reaction with nucleic acid according to claim 8 or its complement.

18. A composition comprising the C-C CKR-1 polypeptide of claim 1 and a pharmaceutically acceptable carrier.

19. A composition comprising the antibody of claim 6 or 7 and a pharmaceutically acceptable carrier.

20. An isolated polypeptide comprising the C-C CKR-1 polypeptide of claim 1 fused to a polypeptide heterologous to the C-C CKR-1 polypeptide.

21. A method for identifying a C-C chemokine receptor as claimed in claim 1 comprising
(a) designing a PCR primer corresponding to a transmembrane region of a seven-transmembrane spanning protein;
(b) priming a PCR reaction with a cDNA substrate from a hematopoietic cell type known to respond to a C-C chemokine;
(c) recovering PCR products from step (b).

22. The method of claim 21, wherein the primer comprises a set of degenerate oligonucleotides encoding the sequence
LNLA(L/V)AD(L/F) (L/G).

23. The method of claim 21, wherein the primer comprises a set of degenerate oligonucleotides encoding the sequence
NP(I/M)(I/L)Y(A/V)(F/V)(I/M/A)GQ.

24. The method of claim 21, wherein the primer comprises a set of degenerate oligonucleotides encoding the sequence
DRYLAIVHA.

25. The method of claim 21, wherein the cell type is cultured human monocytes.

26. A method comprising transforming a host cell with DNA encoding C-C CKR-1 polypeptide of claim 1, culturing the host cell to express the receptor on its surface, harvesting the cells, contacting the cells with a C-C chemokine variant, and determining the biological activity of the variant on the receptor.

27. An assay for an analyte of the C-C CKR-1 polypeptide of claim 1, which assay comprises testing for binding of the analyte to a binding partner, wherein said binding partner is said C-C CKR-1 polypeptide.

28. An assay as claimed in claim 27 which is a competitive assay, a sandwich assay, or a steric inhibition assay.

29. An assay as claimed in claim 27 or claim 28 which uses one or more of the following reagents: labeled analyte analog, immobilized analyte analog, labeled binding partner, immobilized binding partner.

30. An assay as claimed in claim 29 wherein the binding partner is separated from any analyte that remains free in solution.

31. An assay as claimed in claim 30 which uses an analyte analog, wherein either the binding partner or analyte analog is insolubilized before the assay procedure.

32. An assay as claimed in claim 31 which uses an analyte analog, wherein either the binding partner or analyte analog is insolubilized after the assay procedure.

33. An assay as claimed in claim 28 which is a homogeneous competitive assay.

34. An assay as claimed in claim 28 which is a simultaneous sandwich assay.

35. An assay as claimed in claim 28 which is a sequential sandwich assay.

## Patentansprüche

1. Isoliertes C-C-CKR-1-Polypeptid, das (i) die Fähigkeit besitzt, sich an zumindest ein C-C-Chemokin zu binden, und (ii) die in Fig. 1 gezeigte Aminosäuresequenz oder eine Sequenz, die über ihre gesamte Länge zumindest 60 % Sequenzidentität mit der in Fig. 1 gezeigten Sequenz aufweist, umfasst.

2. Polypeptid nach Anspruch 1, das eine Aminosäuresequenz aufweist, die über ihre gesamte Länge zumindest 70 % Sequenzidentität mit der in Fig. 1 gezeigten Sequenz hat.

3. Polypeptid nach Anspruch 2, das die in Fig. 1 gezeigte Aminosäuresequenz aufweist.

4. Polypeptid nach einem der vorangehenden Ansprüche, das die Fähigkeit besitzt, sich an ein aus RANTES, HuMIP-1α, HuMIP-1β oder MCP-1 ausgewähltes C-C-Chemokin zu binden.

5. Zusammensetzung, umfassend das C-C-CKR-1-Polypeptid nach Anspruch 1, worin das C-C-CKR-1-Polypeptid aus einer natürlichen Quelle stammt und worin die Zusammensetzung frei von jeglichem verunreinigendem Polypeptid der Tierspezies ist, die die Quelle des C-C-CKR-1-Polypeptids darstellt.

6. Antikörper, der in der Lage ist, das C-C-CKR-1-Polypeptid nach Anspruch 1 zu binden, das mit keinem anderen Chemokinrezeptor kreuzreagiert.

7. Antikörper nach Anspruch 6, der ein monoklonaler Antikörper ist.

8. Isoliertes Nucleinsäuremolekül, das für das C-C-CKR-1-Polypeptid nach Anspruch 1 kodiert.

9. Nucleinsäuremolekül nach Anspruch 8, das DNA ist und mehr als etwa 25 Basen enthält.

10. Nucleinsäuremolekül nach Anspruch 8, das cDNA ist.

11. Nucleinsäuremolekül nach Anspruch 8, das eine genomische Sequenz ist.

12. Nucleinsäuremolekül nach Anspruch 8, das weiters einen operabel an die Nucleinsäuresequenz gebundenen Promotor umfasst.

13. Nucleinsäuremolekül nach Anspruch 12, worin der Promotor zum C-C-CKR-1-Polypeptid heterolog ist.

14. Expressionsvektor, umfassend die Nucleinsäuresequenz nach Anspruch 8, die operabel an Kontrollsequenzen gebunden ist, die durch eine mit dem Vektor transformierte Wirtszelle erkannt werden.

15. Wirtszelle, die mit dem Vektor nach Anspruch 14 transformiert ist.

16. Verfahren zum Bestimmen der Gegenwart einer C-C-CKR-1-Nucleinsäure, umfassend das Hybridisieren von Nucleinsäure nach Anspruch 8 oder ihres Komplements an eine Testprobennucleinsäure und das Bestimmen der Gegenwart von C-C-CKR-1-Nucleinsäure, die hieran hybridisiert.

17. Verfahren zur Amplifikation einer Nucleinsäurentestprobe, umfassend das Primen einer Nucleinsäure-Polymerasereaktion mit Nucleinsäure nach Anspruch 8 oder ihrem Komplement.

18. Zusammensetzung, umfassend das C-C-CKR-1-Polypeptid nach Anspruch 1 und einen pharmazeutisch annehmbaren Träger.

19. Zusammensetzung, umfassend den Antikörper nach Anspruch 6 oder 7 und einen pharmazeutisch annehmbaren Träger.

20. Isoliertes Polypeptid, umfassend das C-C-CKR-1-Polypeptid nach Anspruch 1, fusioniert an ein Polypeptid, das heterolog zum C-C-CKR-1-Polypeptid ist.

21. Verfahren zur Identifikation eines C-C-Chemokinrezeptors nach Anspruch 1, umfassend:
(a) den Entwurf eines PCR-Primers, der einer Transmembranregion eines sieben Transmembrane umspannenden Proteins entspricht;
(b) das Primen einer PCR-Reaktion mit einem cDNA-Substrat aus einem blutbildenden Zelltyp, der für seine Reaktionsfähigkeit gegenüber einem C-C-Chemokin bekannt ist;
(c) das Gewinnen von PCR-Produkten aus Schritt (b).

22. Verfahren nach Anspruch 21, worin der Primer eine Reihe von degenerierten Oligonucleotiden umfasst, die für die Sequenz
LNLA (L/V) AD (L/F) (L/G) kodieren.

23. Verfahren nach Anspruch 21, worin der Primer eine Reihe von degenerierten Oligonucleotiden umfasst, die für die Sequenz
NP (I/M) (I/L) Y (A/V) (F/V) (I/M/A) GQ kodieren.

24. Verfahren nach Anspruch 21, worin der Primer eine Reihe von degenerierten Oligonucleotiden umfasst, die für die Sequenz
DRYLAIVHA kodieren.

25. Verfahren nach Anspruch 21, worin der Zelltyp kultivierte menschliche Monozyten ist.

26. Verfahren, umfassend das Transformieren einer Wirtszelle mit für das C-C-CKR-1-Polypeptid nach Anspruch 1 kodierender DNA, das Kultivieren der Wirtszelle zur Expression des Rezeptors an ihrer Oberfläche, das Ernten der Zellen, das Kontaktieren der Zellen mit einer C-C-Chemokinvariante und das Bestimmen der biologischen Aktivität der Variante am Rezeptor.

27. Test für einen Analyten des C-C-CKR-1-Polypeptids nach Anspruch 1, worin der Test das Testen auf Bindung des Analyten an einen Bindungspartner umfasst, worin der Bindungspartner das C-C-CKR-1-Polypeptid ist.

28. Test nach Anspruch 27, der ein kompetitiver Test, ein Sandwich-Test oder ein sterischer Hinderungstest ist.

29. Test nach Anspruch 27 oder Anspruch 28, der eines oder mehrere der folgenden Reagenzien verwendet: markiertes Analytanalogon, immobilisiertes Analytanalogon, markierter Bindungspartner, immobilisierter Bindungspartner.

30. Test nach Anspruch 29, worin der Bindungspartner von sämtlichen Analyten getrennt wird, die frei in Lösung bleiben.

31. Test nach Anspruch 30, der ein Analytanalogon verwendet, worin entweder der Bindungspartner oder das Analytanalogon vor dem Testverfahren unlöslich gemacht wird.

32. Test nach Anspruch 31, der ein Analytanalogon verwendet, worin entweder der Bindungspartner oder das Analytanalogon nach dem Testverfahren unlöslich gemacht wird.

33. Test nach Anspruch 28, der ein homogener kompetitiver Test ist.

34. Test nach Anspruch 28, der ein gleichzeitiger Sandwich-Test ist.

35. Test nach Anspruch 28, der ein sequenzieller Sandwich-Test ist.

## Revendications

1. Polypeptide C-C CKR-1 isolé qui possède (i) la faculté de se lier au moins à une chimiokine C-C, et (ii) la séquence d'aminoacides décrite sur la figure 1 ou une séquence identique au moins à 60% sur toute sa longueur avec la séquence de la figure 1.

2. Polypeptide selon la revendication 1, qui possède une séquence d'aminoacides identique au moins à 70% sur toute sa longueur avec la séquence de la figure 1.

3. Polypeptide selon la revendication 2, qui possède la séquence d'aminoacides décrite sur la figure 1.

4. Polypeptide selon l'une quelconque des revendications précédentes, qui possède la capacité de se lier à une chimiokine C-C choisie parmi :
RANTES, HuMIP-1α, HuMIP-1β ou MCP-1.

5. Composition comprenant le polypeptide C-C CKR-1 selon la revendication 1, dans laquelle le polypeptide C-C CKR-1 est dérivé d'une source naturelle, et ladite composition est dépourvue d'un polypeptide contaminant de l'espèce animale qui est la source du polypeptide C-C CKR-1.

6. Anticorps qui est capable de se lier au polypeptide C-C CKR-1 selon la revendication 1, qui ne fait pas de réaction croisée avec un autre récepteur de chimiokine.

7. Anticorps selon la revendication 6, qui est un anticorps monoclonal.

8. Molécule d'acide nucléique isolée codant pour le polypeptide C-C CKR-1 selon la revendication 1.

9. Molécule d'acide nucléique selon la revendication 8, qui est un ADN et qui contient plus d'environ 25 bases.

10. Molécule d'acide nucléique selon la revendication 8, qui est un ADNc.

11. Molécule d'acide nucléique selon la revendication 8, qui est une séquence génomique.

12. Molécule d'acide nucléique selon la revendication 8, comprenant en outre un promoteur lié opérationnellement à la séquence d'acide nucléique.

13. Molécule d'acide nucléique selon la revendication 12, dans laquelle le promoteur est hétérologue vis-à-vis du polypeptide C-C CKR-1.

14. Vecteur d'expression comprenant la séquence d'acide nucléique selon la revendication 8, opérationnellement lié à des séquences de contrôle reconnues par une cellule hôte transformée avec le vecteur.

15. Cellule hôte transformée avec le vecteur selon la revendication 14.

16. Procédé de détermination de la présence d'un acide nucléique C-C CKR-1, comprenant l'hybridation d'un acide nucléique selon la revendication 8 ou de son complément à l'acide nucléique d'un échantillon d'essai et la détermination de la présence de l'acide nucléique C-C CKR-1 qui s'hybride à celui-ci.

17. Procédé d'amplification d'un échantillon d'essai d'acide nucléique comprenant l'amorçage d'une réaction de polymérase d'acide nucléique avec l'acide nucléique selon la revendication 8 ou son complément.

18. Composition comprenant le polypeptide C-C CKR-1 selon la revendication 1 et un support pharmaceutiquement acceptable.

19. Composition comprenant l'anticorps selon la revendication 6 ou 7 et un support pharmaceutiquement acceptable.

20. Polypeptide isolé comprenant le polypeptide C-C CKR-1 selon la revendication 1 condensé à un polypeptide hétérologue vis-à-vis du polypeptide C-C CKR-1.

21. Procédé d'identification d'un récepteur de chimiokine C-C selon la revendication 1, comprenant :
(a) la conception d'une amorce de PCR correspondant à une région transmembranaire d'une protéine couvrant sept régions transmembranaires ;
(b) l'amorçage d'une réaction de PCR avec un substrat d'ADNc provenant d'un type de cellule hématopoïétique connu pour répondre à une chimiokine C-C ;
(c) la récupération des produits de PCR provenant de l'étape (b).

22. Procédé selon la revendication 21, dans lequel l'amorce comprend un ensemble d'oligonucléotides dégénérés codant pour la séquence
LNLA (L/V) AD (L/F) (L/G) .

23. Procédé selon la revendication 21, dans lequel l'amorce comprend un ensemble d'oligonucléotides dégénérés codant pour la séquence
NP (I/M) (I/L) Y (A/V) (F/V) (I/M/A) GQ.

24. Procédé selon la revendication 21, dans lequel l'amorce comprend un ensemble d'oligonucléotides dégénérés codant pour la séquence
DRYLAIVHA.

25. Procédé selon la revendication 21, dans lequel le type de cellule consiste en monocytes humains cultivés.

26. Procédé comprenant la transformation d'une cellule hôte avec un ADN codant pour un polypeptide C-C CKR-1 selon la revendication 1, la culture de la cellule hôte afin d'exprimer le récepteur à sa surface, la récolte des cellules, la mise en contact des cellules avec un variant de chimiokine C-C, et la détermination de l'activité biologique du variant sur le récepteur.

27. Analyse d'un analyte du polypeptide C-C CKR-1 selon la revendication 1, ladite analyse comprenant un test de liaison de l'analyte à un partenaire de liaison, dans laquelle ledit partenaire de liaison est ledit polypeptide C-C CKR-1.

28. Analyse selon la revendication 27, qui est une analyse par compétition, une analyse de type sandwich ou une analyse par inhibition stérique.

29. Analyse selon la revendication 27 ou la revendication 28, qui utilise un ou plusieurs des réactifs suivants : un analogue marqué de l'analyte, un analogue immobilisé de l'analyte, un partenaire de liaison marqué, un partenaire de liaison immobilisé.

30. Analyse selon la revendication 29, dans laquelle le partenaire de liaison est séparé de tout analyte qui reste libre en solution.

31. Analyse selon la revendication 30, qui utilise un analogue de l'analyte, dans laquelle soit le partenaire de liaison, soit l'analogue de l'analyte est rendu insoluble avant la mise en oeuvre de l'analyse.

32. Analyse selon la revendication 31, qui utilise un analogue de l'analyte, dans laquelle soit le partenaire de liaison, soit l'analogue de l'analyte est rendu insoluble après la mise en oeuvre de l'analyse.

33. Analyse selon la revendication 28, qui est une analyse par compétition homogène.

34. Analyse selon la revendication 28, qui est une analyse de type sandwich simultanée.

35. Analyse selon la revendication 28, qui est une analyse de type sandwich séquentielle.
